# EUROPEAN PATENT APPLICATION

(11) **EP 1 738 735 A1**
(43) Date of publication of application: **03.01.2007**
(21) Application number: 05734096.0
(22) Date of filing: 19.04.2005
(51) Int. Cl.: A61G 13/00, A61B 1/00

(54) **MEDICAL BED**

(30) Priority: 21.04.2004 JP 2004125760
(71) Applicant: Olympus Corporation, Tokyo 151-0072 (JP)
(72) Inventor: NOGUCHI, Toshiaki, Olympus Corporation, Shibuya-ku, Tokyo 1510072 (JP); ONODA, Fumiyuki Olympus Corporation, Tokyo 151-0072 (JP); TANIGUCHI, Akira Olympus Corporation, Tokyo 151-0072 (JP); UCHIMURA, Sumihiro Olympus Corporation, Tokyo 151-0072 (JP); SUZUKI, Katsuya Olympus Corporation, Tokyo 151-0072 (JP)
(74) Representative: von Hellfeld, Axel
(86) International application number: PCT/JP2005/007457
(87) International publication number: WO 2005/102245

(57) **Abstract**

A medical bed in which an endoscope after use can be easily tidied up is provided. A medical bed 2 of the present invention constitutes a medical bed having a laying table 2a on which a patient is laid for inspection or treatment with an endoscope, and it has a tray housing portion 2b for housing an endoscope tray 39 in which an endoscope 3 is housed.

## Description

### Technical Field

The present invention relates to a medical bed used for inspection and the like using an endoscope inserted into a body cavity.

### Background Art

Endoscopes are widely used in the medical and industrial fields recently. Particularly, endoscopes used in the medical field are capable of observation of organs in a body cavity by inserting an elongated insertion portion into the body cavity or various treatments using a treatment instrument inserted into an insertion channel of the treatment instrument at need.

Since the endoscope in the medical field is used by being inserted into a body cavity for the purpose of inspection and treatment, contaminated endoscopes after use are washed and disinfected. Therefore, operators, nurses and the like carry out by hand the contaminated endoscopes in a bucket, a tray or the like placed in an operation room for washing and the like after an inspection or the like so that the endoscopes may be washed or the like (See Japanese Unexamined Patent Application Publication No. 8-110479, for example).

However, when the contaminated endoscope is to be put in the bucket, the tray or the like, the operator or the like should carefully put the endoscope in the bucket or the like and carry out the bucket or the like lest the endoscopes should touch the peripheral devices. Thus, the operator or the like should take utmost care till the operator places the endoscope in the bucket or the like after the inspection and the like, and tidying up is a cumbersome work.

The present invention is made in view of the above points and its object is to provide an operation system which can easily tidy up endoscopes after use.

### Disclosure of Invention

### Means for Solving the Problems

The medical bed according to the present invention comprises a laying table on which a patient is placed for inspection or treatment using an endoscope and a tray housing portion for housing an endoscope housing tray in which the endoscope is housed.

### Brief Description of the Drawings

Fig. 1 is a schematic block diagram of an endoscope system provided with an embodiment of the present invention.
Figs. 2A to 2C show data communication forms according to the embodiment of the present invention, in which Fig. 2A is a view for explaining a wireless method.
Fig. 2B is a diagram for explaining a wired method.
Fig. 2C is a diagram for explaining an optical communication method.
Fig. 3 is a diagram showing an outline configuration of an endoscope of the embodiment of the present invention.
Fig. 4 is a perspective view showing an entire configuration of the endoscope system provided with the embodiment of the present invention.
Fig. 5 is a perspective view showing a specific appearance form of a peripheral portion of a unit as air-supply/water-supply/suction device (hereinafter referred to as AWS unit).
Fig. 6A is a perspective view showing a state where a freely detachable AWS adapter is mounted to the AWS unit.
Fig. 6B is a perspective view showing a state where the freely detachable AWS adapter is removed from the AWS unit.
Figs. 7A to 7E show the AWS adapter in detail and Fig. 7A is a front view of an AWS adapter 42.
Fig. 7B is a left side view of the AWS adapter.
Fig. 7C is a right side view of the AWS adapter 42.
Fig. 7D is a sectional view along A-A' line in Fig. 7A.
Fig. 7E is a sectional view along B-B' line in Fig. 7A.
Fig. 8 is a diagram showing a structure of the AWS adapter.
Fig. 9 is an entire view showing a detailed configuration of the endoscope of the embodiment of the present invention.
Fig. 10A is a view for explaining a function of contraction of a conductive polymer artificial muscle (EPAM) and is a view showing EPAM in the state before voltage application.
Fig. 10B is a view showing the EPAM in the state where voltage is applied to the EPAM.
Fig. 10C is a graph for explaining outline characteristics of the EPAM and is a graph showing a relation between the applied voltage and a distortion quantity.
Fig. 11 is a view showing a track ball and the like provided at an operation portion in view on arrow C of Fig. 9.
Fig. 12 is a circuit diagram showing a configuration of a contactless transmission portion to which a base end of a tube unit is freely detachably connected in a contactless manner to the operation portion main body.
Fig. 13 is a block diagram showing a configuration of an electric system in components provided inside the endoscope.
Fig. 14 is a block diagram showing a configuration of an electric system of a main part of an endoscope system control device.
Fig. 15 is a block diagram showing a configuration of an electric system of the AWS unit.
Fig. 16A is a diagram showing a specific example of a typical display of a monitor display surface of an observation monitor.
Fig. 16B is a diagram showing a specific example of menu display of the monitor display surface of the observation monitor.
Fig. 16C is a diagram showing a display example for selecting and setting a function to be allocated to a scope switch.
Fig. 17 is a flowchart showing an operation content of start processing of the AWS unit.
Fig. 18 is a flowchart showing an operation content of start processing of the endoscope.
Fig. 19 is a flowchart showing an operation content of image capturing control processing.
Fig. 20 is a flowchart showing an operation content of control processing of air/water supply.
Fig. 21 is a flowchart showing control processing of an angle operation.
Fig. 22 is a flowchart showing a control operation for rigidity changing operation.
Fig. 23A is an operational explanatory view showing a setting operation of rigidity changing and an UPD image corresponding to that operation.
Fig. 23B is an operational explanatory view showing a setting operation of rigidity changing and an UPD image corresponding to that operation.
Fig. 23C is an operational explanatory view showing a setting operation of rigidity changing and an UPD image corresponding to that operation.
Fig. 23D is an operational explanatory view showing a setting operation of rigidity changing and an UPD image corresponding to that operation.
Fig. 24 is a flowchart showing a processing content on the endoscope side in human interface.
Fig. 25 is a flowchart showing a processing content on the endoscope system control device side in human interface.
Fig. 26 is a perspective view showing a configuration of a variation of the endoscope system.

### Best Mode for Carrying Out the Invention

Embodiments of the present invention will be described referring to the attached drawings.

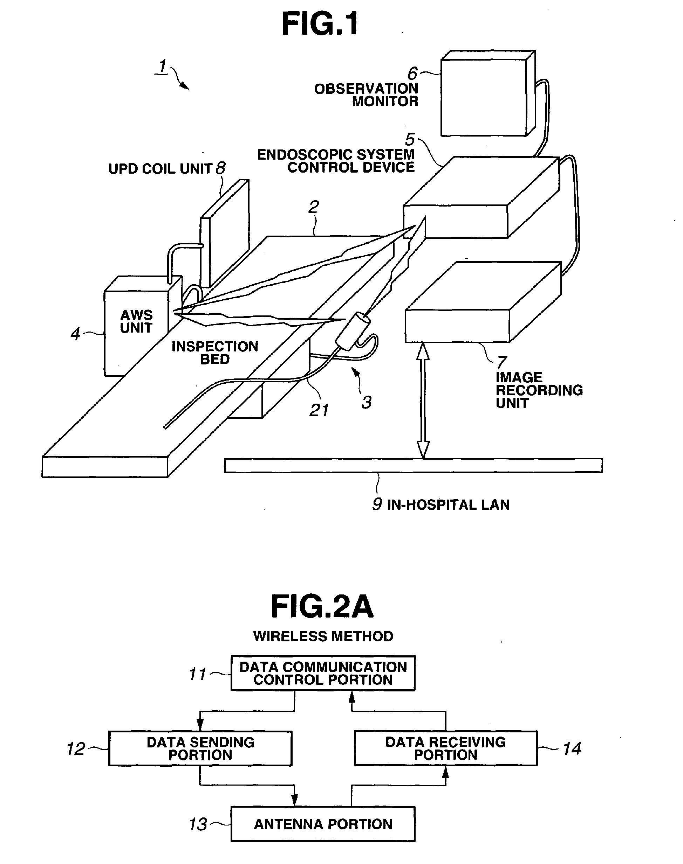

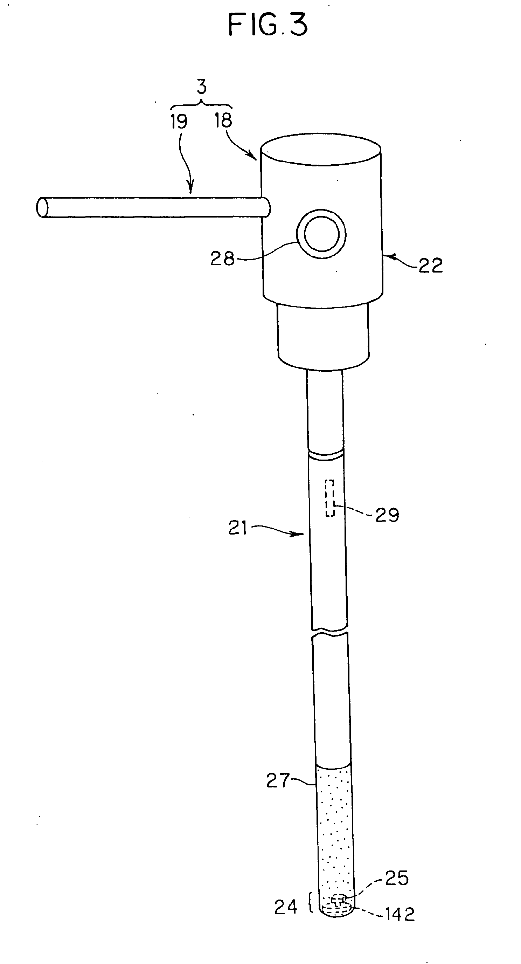

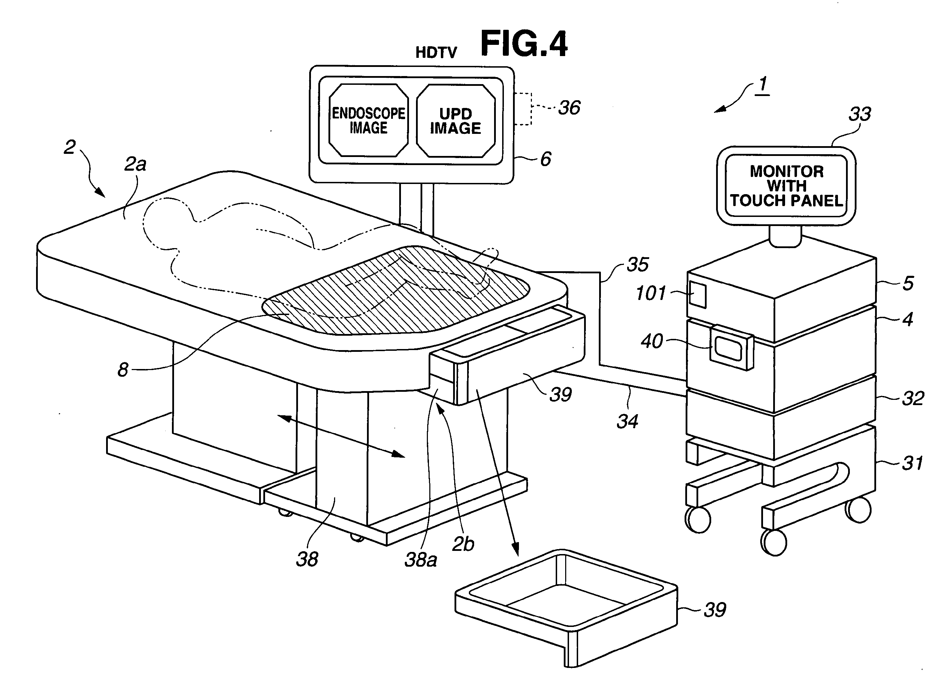

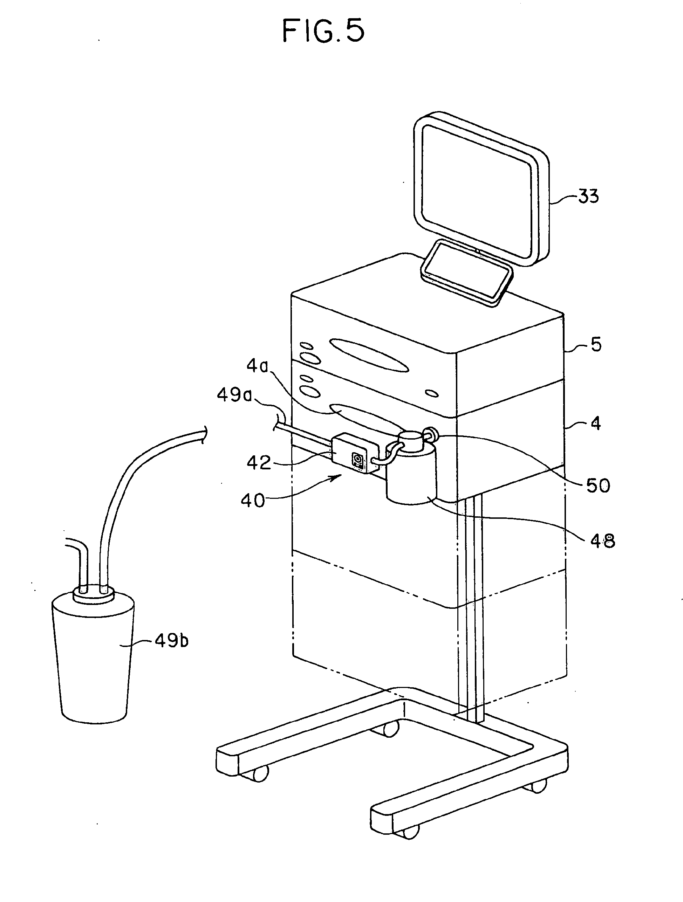

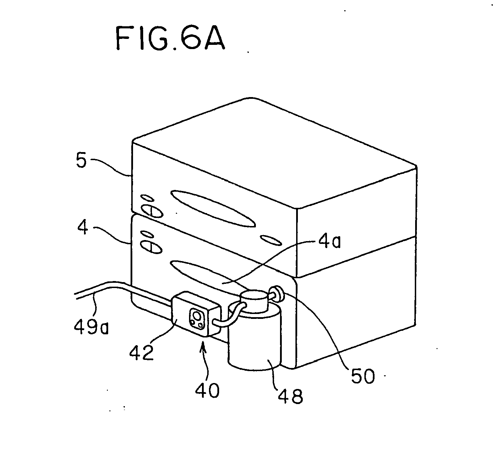

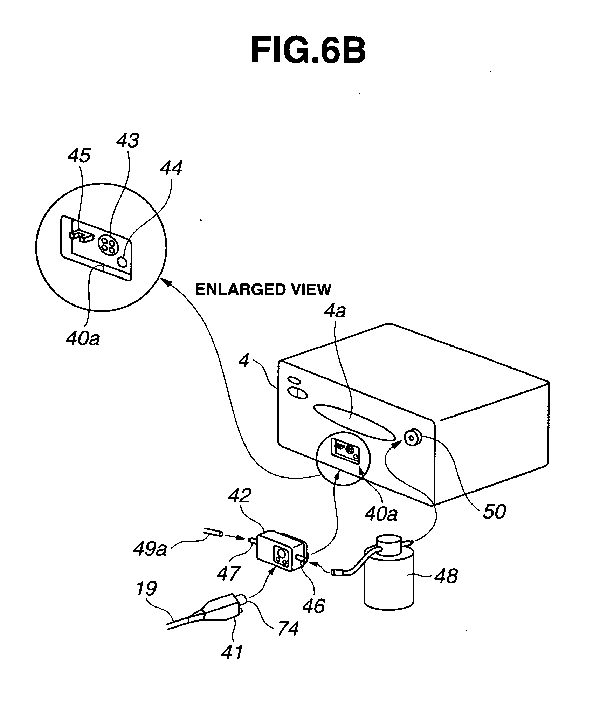

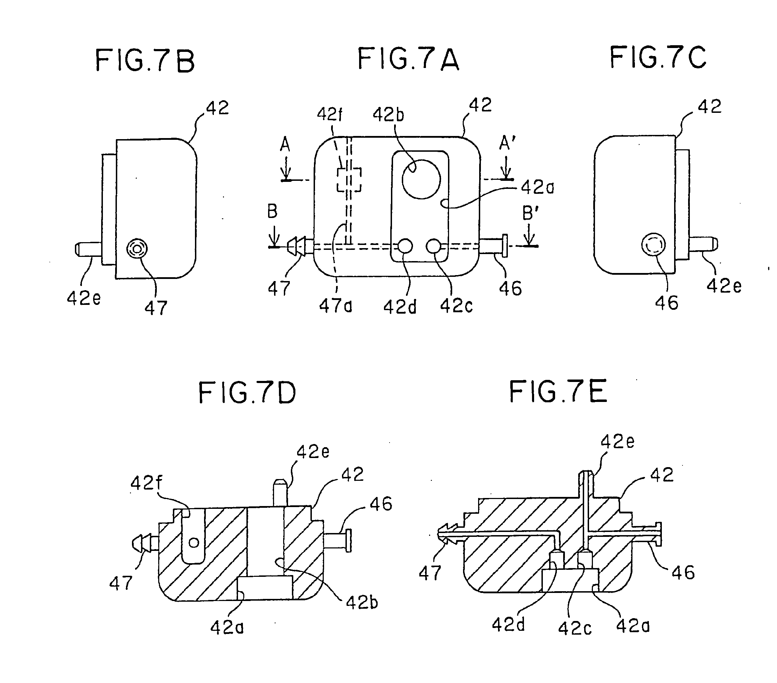

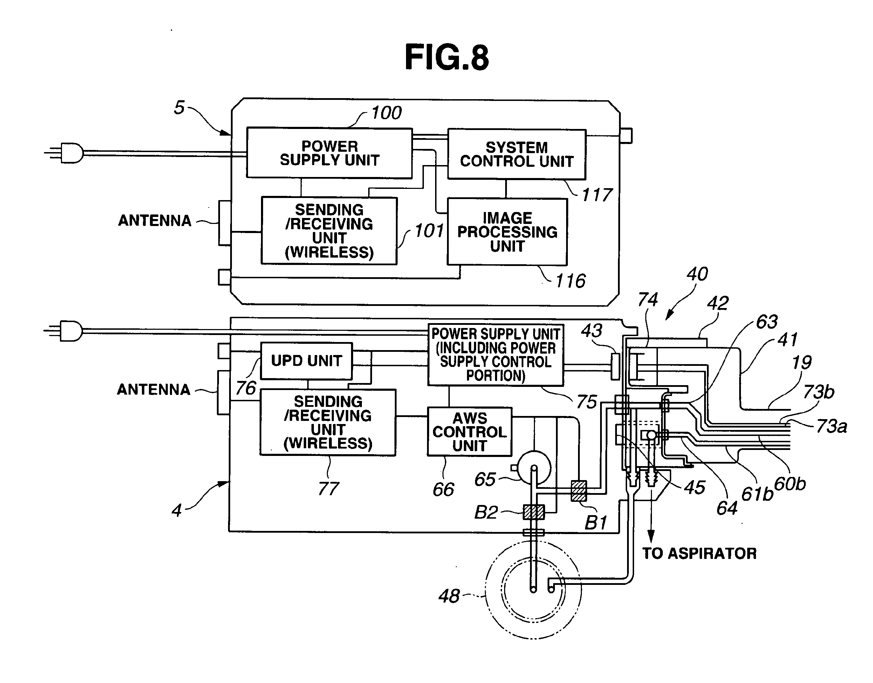

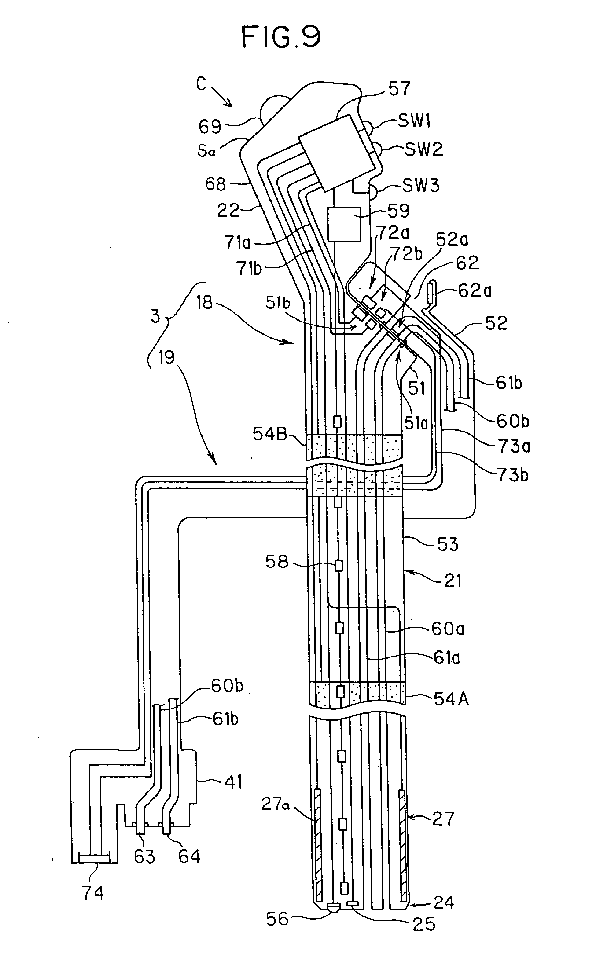

Figs. 1 to 26 relate to an embodiment of the present invention, in which Fig. 1 shows an entire configuration of an endoscope system provided with a medical bed according to the embodiment of the present invention, Figs. 2A to 2C show data communication forms, Fig. 3 shows an outline configuration of an endoscope of the present invention, Fig. 4 shows an entire configuration of the endoscope system provided with the embodiment, Fig. 5 shows a specific appearance form of a peripheral portion of a unit as air-supply/water-supply/suction device (hereinafter referred to as AWS unit), Figs. 6A and 6B show states where a detachable AWS adapter is mounted to the AWS unit and where the freely detachable AWS adapter is removed from the AWS unit, Figs. 7A to 7E show the structure of the AWS adapter, Fig. 8 shows internal structures of the endoscope system control device and the AWS unit, and Fig. 9 shows a detailed configuration of the endoscope of the embodiment.

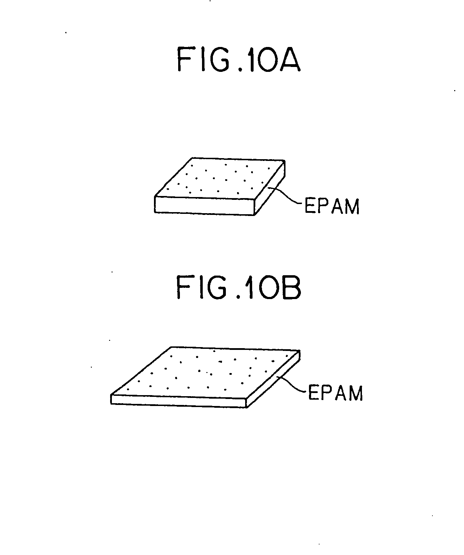

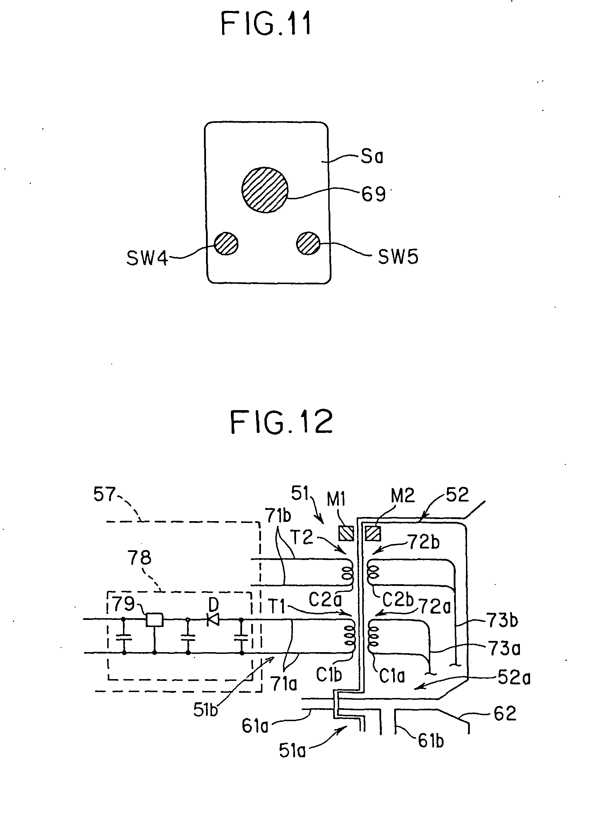

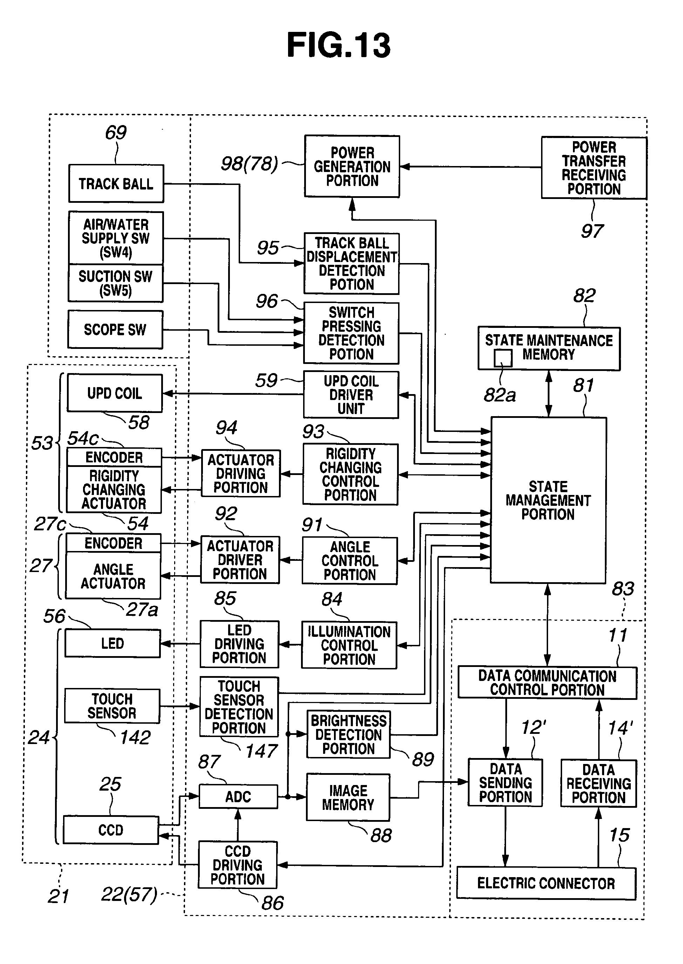

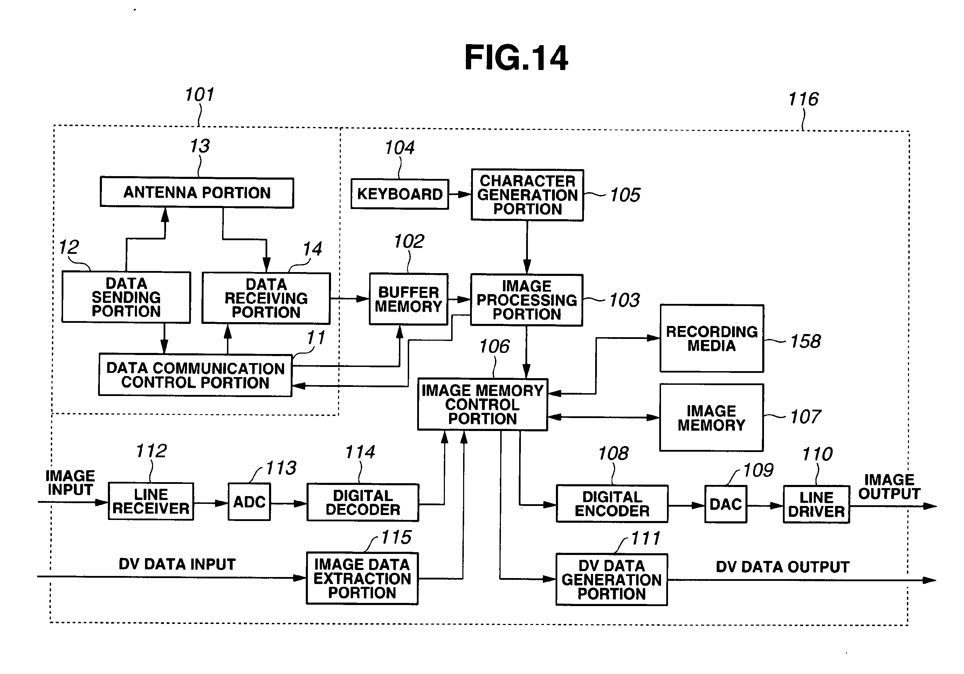

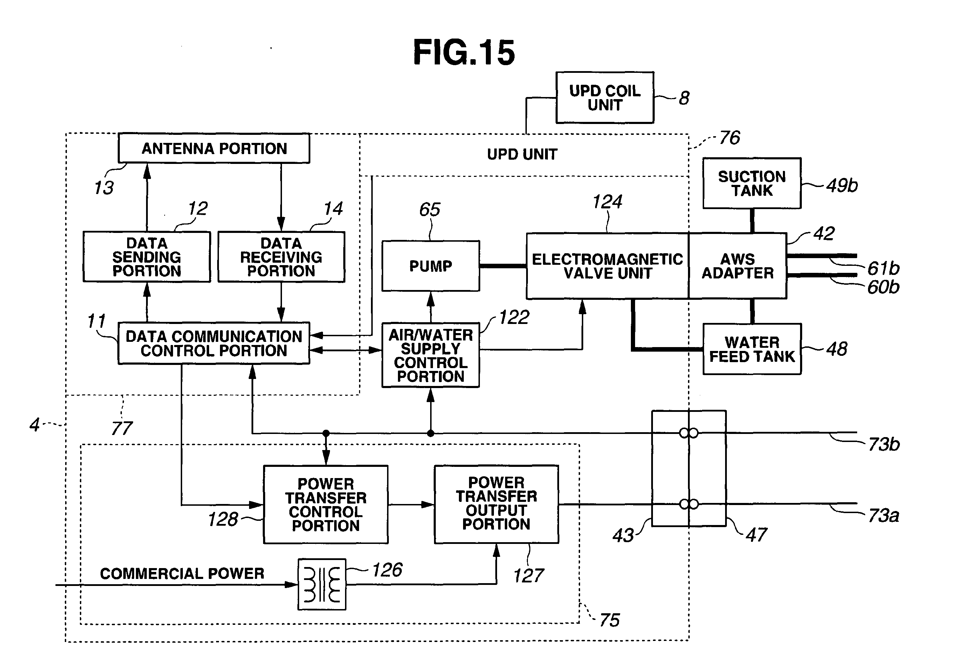

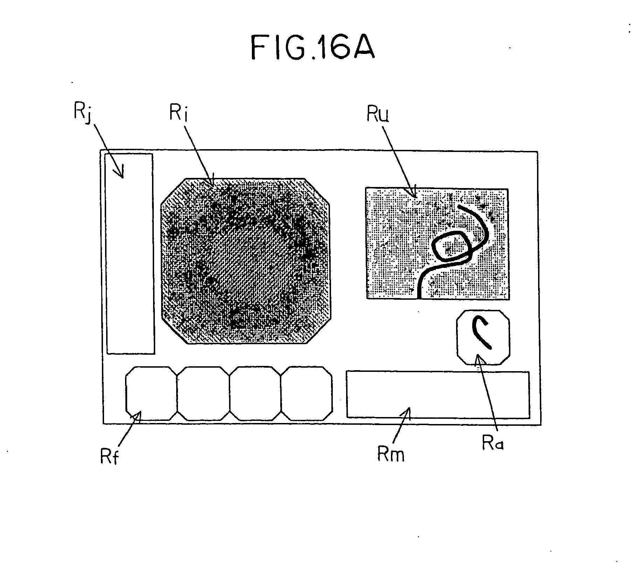

Also, Figs. 10A to 10C show outline characteristics of a conductive polymer artificial muscle (EPAM) used for a member for angle and a member for rigidity changing, Fig. 11 shows a track ball and the like provided at an operation portion in view on arrow C of Fig. 9, Fig. 12 shows a configuration of a contactless transfer portion where a base end of a tube unit is freely detachably connected to the operation portion main body in a contactless manner, Fig. 13 shows a configuration of an electric system in components provided in the endoscope, Fig. 14 shows a configuration of an electric system of main part of the endoscope system control device, Fig. 15 shows a configuration of an electric system of the AWS unit and Figs. 16A to 16C show specific examples of typical display examples and menu display of a monitor display surface of an observation monitor.

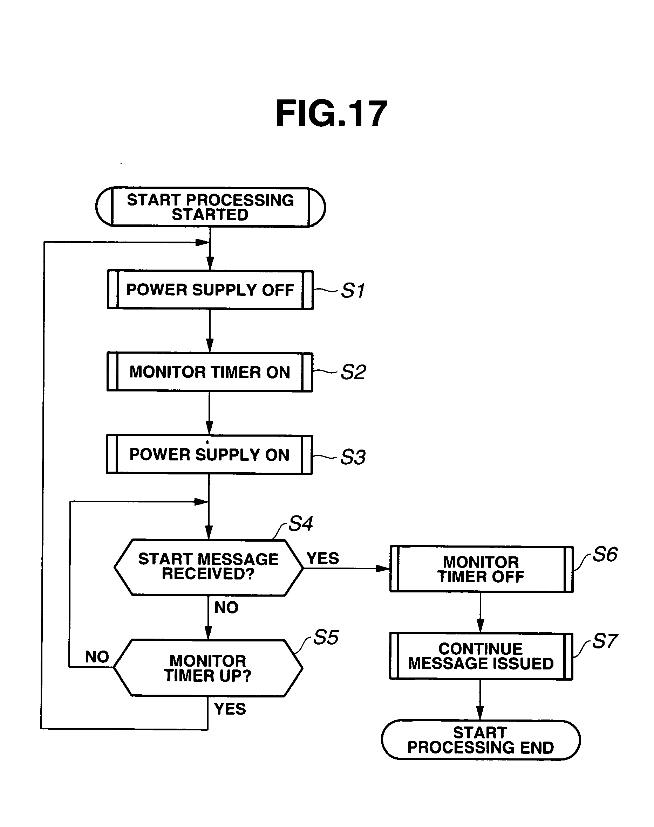

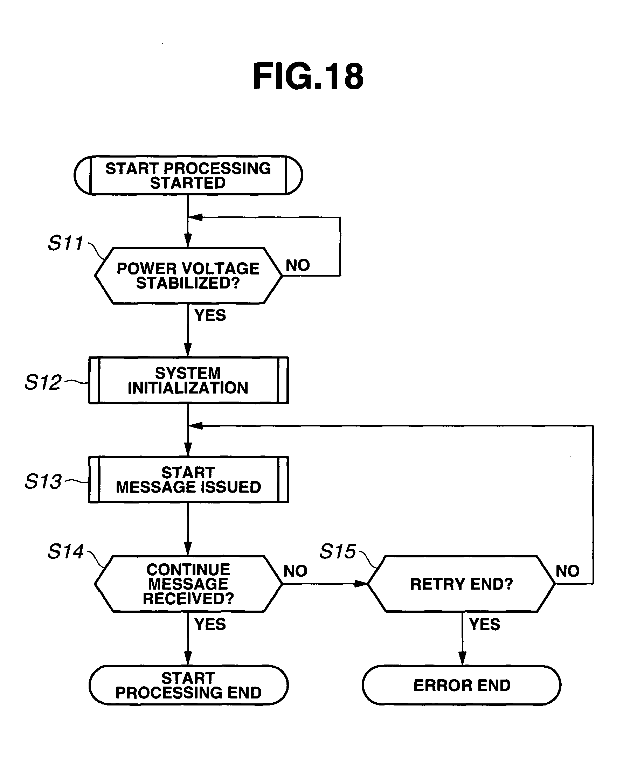

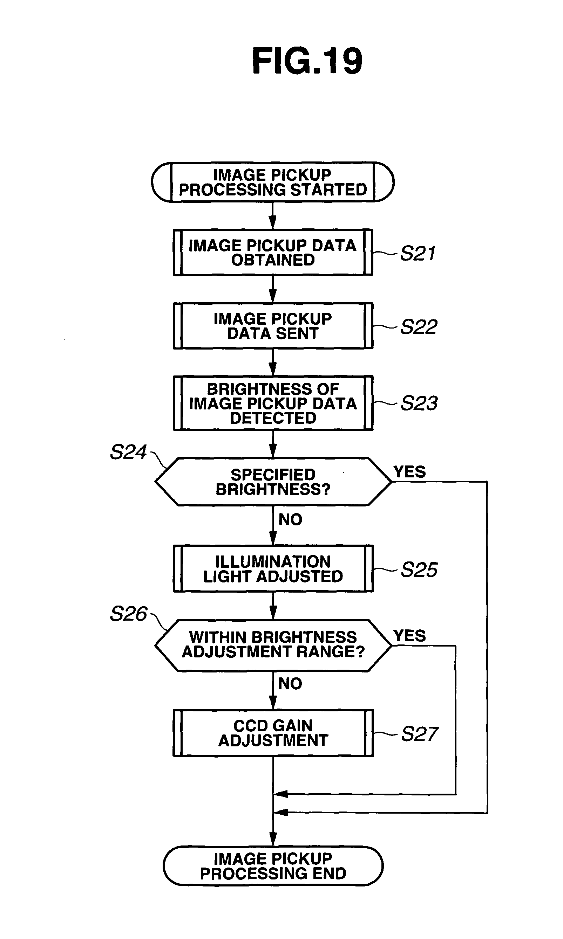

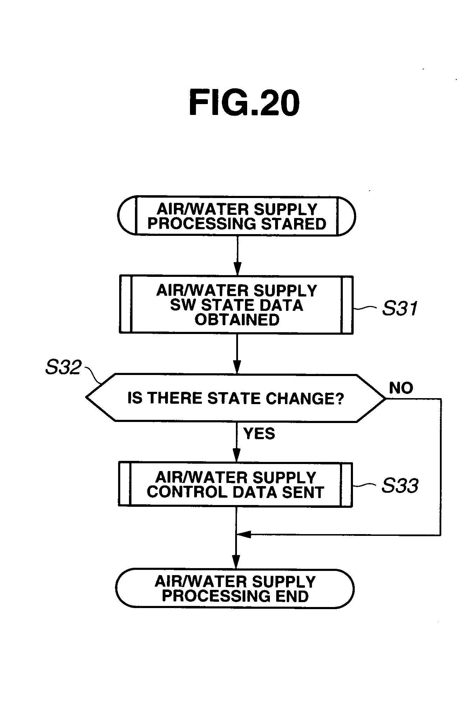

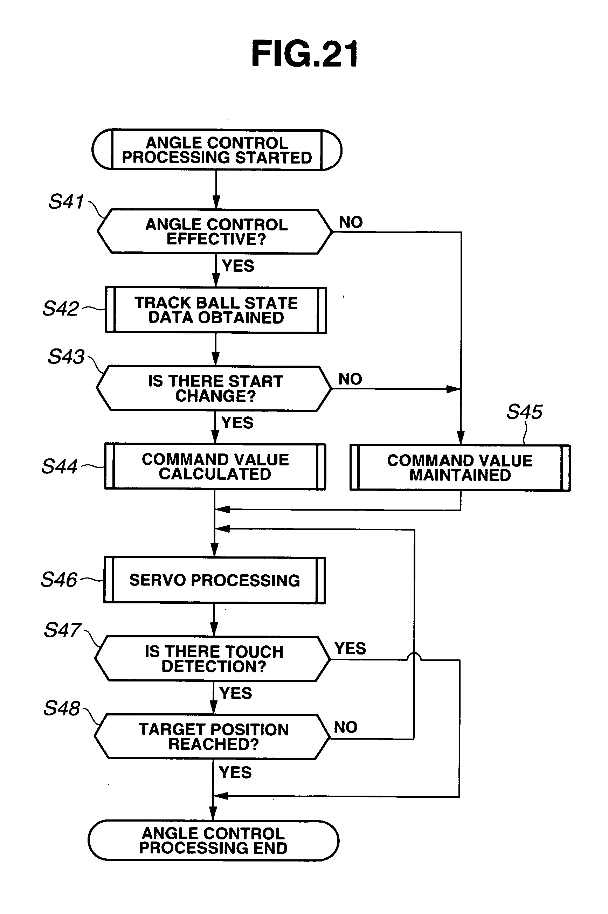

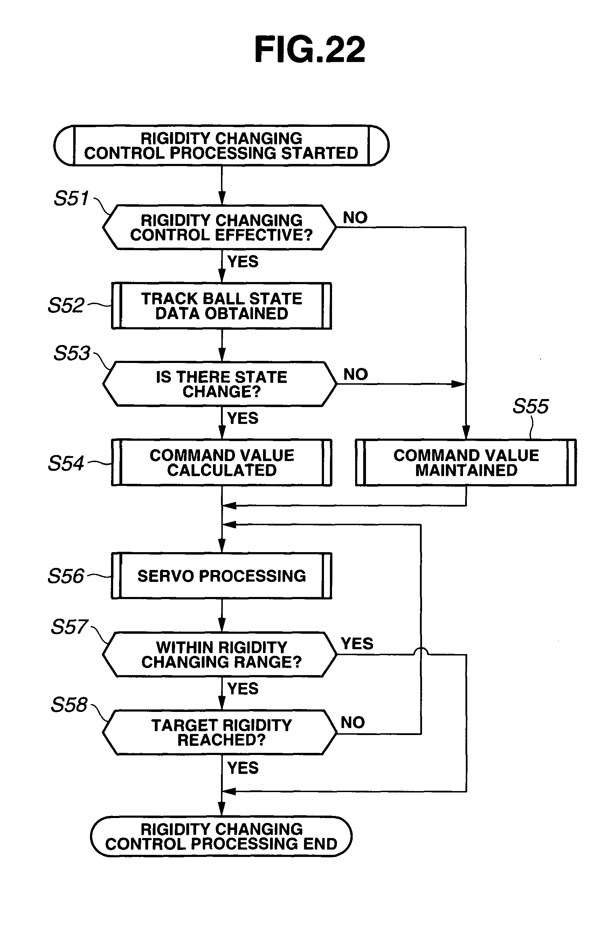

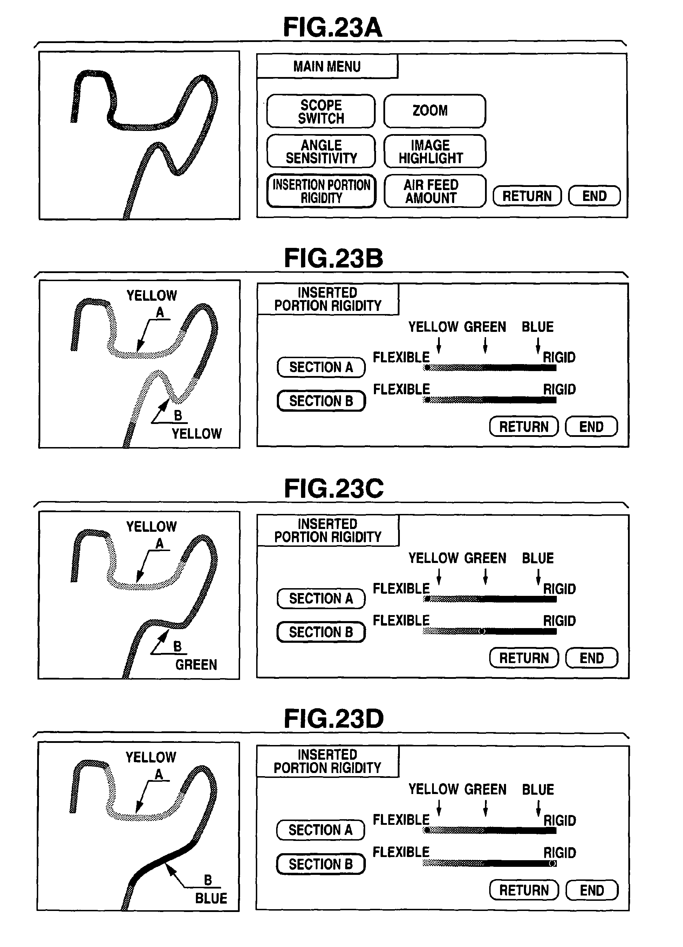

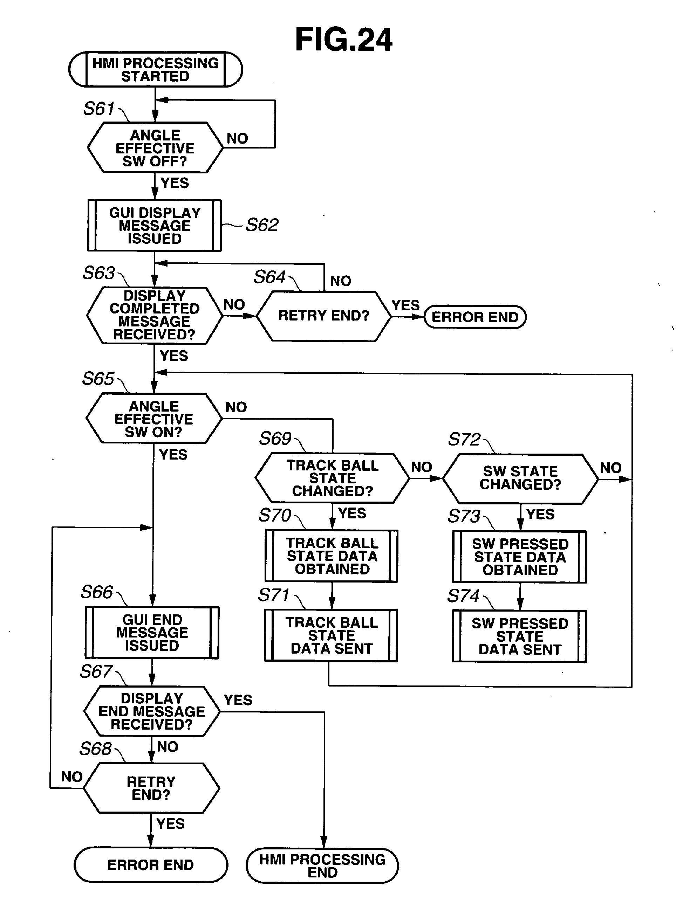

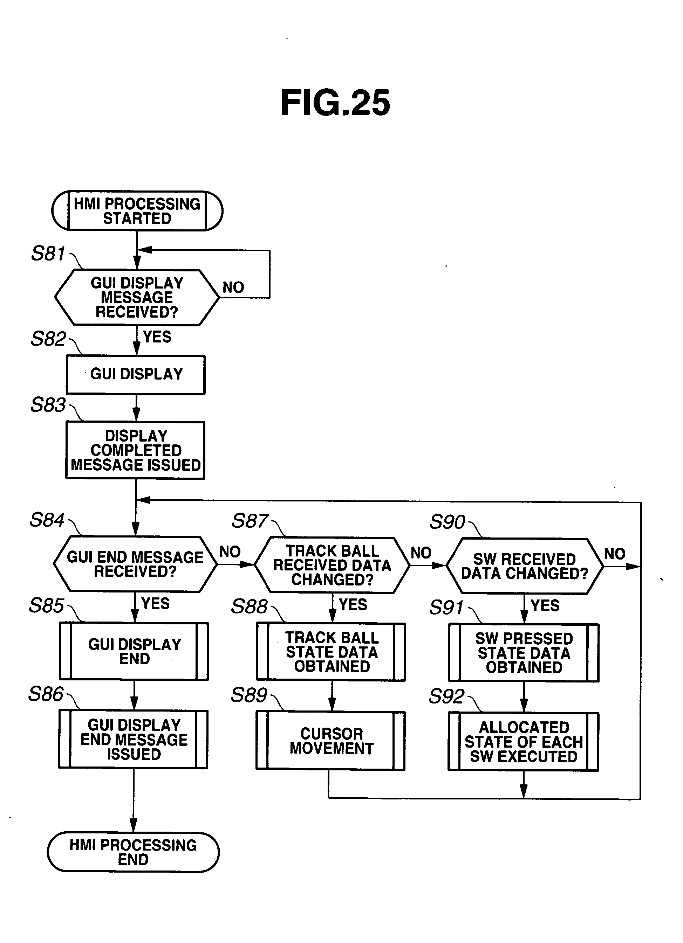

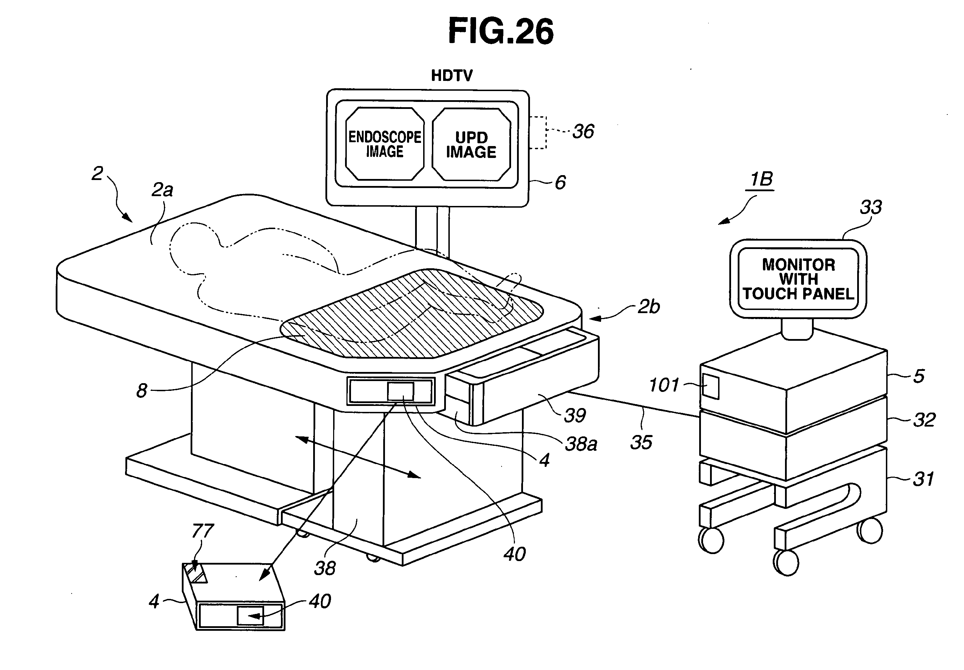

Moreover, Fig. 17 shows an operation content of start processing of the AWS unit, Fig. 18 shows an operation content of start processing of the endoscope, Fig. 19 shows an operation content of image capturing control processing, Fig. 20 shows an operation content of control processing of air/water supply, Fig. 21 shows control processing of an angle operation, Fig. 22 shows a control operation for rigidity changing operation, Figs. 23A to 23D show setting operations of rigidity changing and UPD images corresponding to those operations, Figs. 24 and 25 show respective processing contents on the endoscope side and the endoscope system control device side in human interface, and Fig. 26 shows a configuration of a variation of the endoscope system.

Before describing specific configurations of the embodiment of the present invention, an outline configuration of the embodiment of the present invention will be described referring to Figs. 1 to 3.

As shown in Fig. 1, an endoscope system 1 provided with a medical bed according to the embodiment of the present invention has a flexible endoscope 3 for conducting an endoscope inspection (hereinafter, also referred to as a scope) by being inserted into a body cavity of a patient, not shown, lying down on an inspection bed 2, a unit (AWS unit) 4 as an air-supply/water supply/suction device provided with air supply, water supply and suction functions to which this endoscope 3 is connected, an endoscope system control device 5 for performing signal processing of image pickup device incorporated in the endoscope 3 and control processing and the like of various operating means provided at the endoscope 3, and an observation monitor 6 constituting a liquid crystal monitor or the like for displaying an image signal generated by the endoscope system control device 5.

This endoscope system 1 comprises: an image recording unit 7 for filing or the like a digital image signal, which is for example, generated by the endoscope system control device 5; and an UPD coil unit 8 constituting a device for insertion form detection for detecting the position of each UPD coil by receiving a signal of an electromagnetic field generated by the UPD coil and displaying the form of the insertion portion of the endoscope 3 in a case that a coil for form detection (hereinafter abbreviated as UPD coil) is connected to the AWS unit 4 with being arranged in an insertion portion of the endoscope 3.

Moreover, the image recording unit 7 is connected to a LAN 9 in a hospital at which this endoscope system 1 is provided so that images and the like filed in the image recording unit 7 can be referred to by each terminal device connected to this LAN 9 in a wired or a wireless manner.

Also, as shown in Fig. 1, the AWS unit 4 and the endoscope system control device 5 send/receive information, that is, data in the wireless manner. In Fig. 1, the endoscope 3 is connected to the AWS unit 4 by a cable, but it may send/receive information, that is, data (bidirectional transmission) in the wireless manner. Also, the endoscope system control device 5 may send/receive information to/from the endoscope 3 in the wireless manner.

Figs. 2A to 2C show three methods in a sending/receiving unit (communication portion) for sending/receiving data between the unit and the device, the endoscope 3 and the unit or the devices, in the endoscope system 1. In Fig. 2A, a case of the AWS unit 4 and the endoscope system control device 5 is described as a specific example.

Fig. 2A is a diagram for explaining a wireless method, in which data is modulated through a data sending portion 12 and sent from an antenna portion 13 by a data communication control portion 11 built in the AWS unit 4 to the endoscope system control device 5 wirelessly.

Also, the AWS unit 4 receives data sent from the endoscope system control device 5 in the wireless manner at the antenna portion 13 and demodulates it by a data receiving portion 14 and send the data to the data communication control portion 11. In this embodiment, when data is to be sent by the wireless method, a wireless LAN with the maximum data communication rate of 54 Mbps is formed based upon the IEEE802.11 g standard, for example.

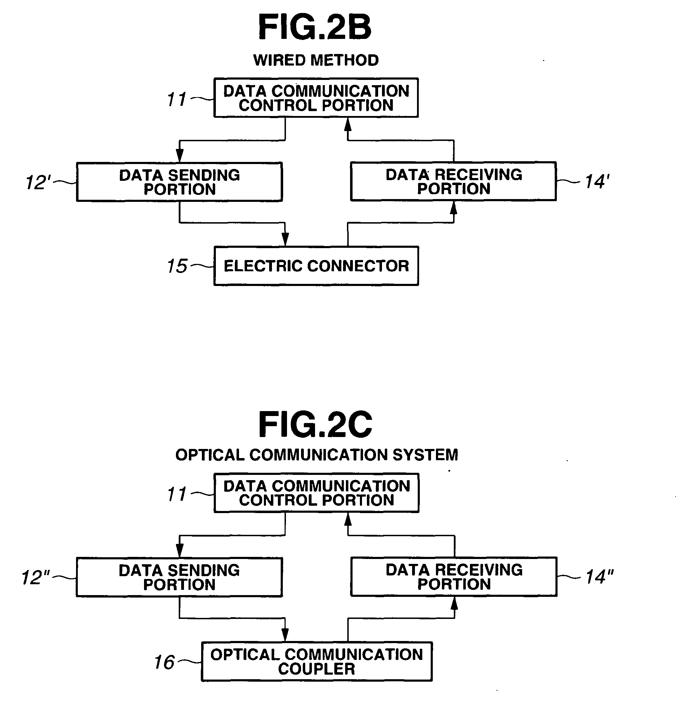

Fig. 2B is a diagram for explaining a wired method, and a case of data transmission between the endoscope 3 and the AWS unit 4 is explained as a specific example. Data is sent from an electric connector 15 through a data sending portion 12' by the data communication control portion 11 built in the endoscope 3 in the wired manner to the AWS unit 4. Also, the data sent from the AWS unit 4 is sent to the data communication control portion 11 through the electric connector 15 and a data receiving portion 14'.

Fig. 2C is a diagram for explaining the optical communication method, and a case of data transmission between the AWS unit 4 and the endoscope system control device 5 is described as a specific example. The data communication control portion 11 built in the AWS unit 4 is connected to an optical communication coupler 16 provided at this AWS unit 4 through a data sending portion 12" for data sending of optical communication and a data receiving portion 14" for data receiving through an optical communication coupler on the endoscope system control device 5 side.

Fig. 3 shows an outline configuration of the endoscope 3 of this embodiment. This embodiment 3 comprises an endoscope main body 18 and a disposable type, for example, tube unit 19 freely detachably connected to the endoscope main body 18. The tube unit 19 has its diameter thinner than that of a conventional universal cable and comprises in this embodiment only two pipeline tubes 63, 64, a power line 73a and a signal line 73b.

The endoscope main body 18 has a flexible insertion portion 21 to be inserted into a body cavity and an operation portion 22 provided at a rear end of this insertion portion 21, and to this operation portion 22, the proximal end of the tube unit 19 is freely detachably connected.

Also, at a tip portion 24 of the insertion portion 21, an image pickup unit using a CCD (Charge Coupled Device) 25 with a variable gain inside an image pickup device is arranged. Moreover, at the tip portion 24, a touch sensor is provided for detecting a state where the tip portion 24 is brought into contact (pressure contact) with an internal wall or the like in the body cavity.

Also, at the back end of the tip portion 24, a curved portion 27 which can be curved with a small capacity is provided, and the curved portion 27 can be curved by operating an angle/remote control operator 28 provided at the operation portion 22. This angle/remote control operator 28 is capable of angle operation (curving operation) and a remote control operation and the like such as an operation of air/water supply, suction and the like and a remote control operation (specifically, freeze instruction operation and release instruction operation) to the endoscope system control device 5 and the like. Moreover, a portion whose rigidity is changeable is formed at the insertion portion 21 so as to smoothly insert and the like.

Also, in the insertion portion 21, a washing level detection portion 29 is provided so that a washing level and the like of the pipeline can be detected.

Next, more specific configuration of the endoscope system 1 will be described referring to Fig. 4.

The observation monitor 6 constituted of a liquid crystal monitor and the like is arranged at a side face of the inspection bed 2, which is a medical bed. And on a cart 31 freely movably arranged in the vicinity of one end of the inspection bed 2 in the longitudinal direction, the endoscope system control device 5, the AWS unit 4, and an image file/LAN/electric knife/ultrasonic unit (noted by simplifying an image file unit, a wireless LAN or a wired LAN, an electric knife device, an ultrasonic unit and the like) 32 are arranged, and a monitor 33 with a touch panel is arranged at the uppermost part.

Also, on the side of a housing portion 2b of a scope tray 39, which is a tray for an endoscope for placing the endoscope on an upper face portion of a laying table 2a where the patient is laid down in the inspection bed 2, the UPD coil unit 8 is embedded. This UPD coil unit 8 is connected to the AWS unit 4 by an UPD cable 34.

In this embodiment, the AWS unit 4 and the endoscope system control device 5 send/receive data by wireless sending/receiving units 77, 101 as shown in Fig. 8, for example. Also, as shown in Fig. 4, the observation monitor 6 is connected to a connector for monitor of the endoscope system control device 5 by a monitor cable 35.

As shown in Fig. 4, sending/receiving units 101, 36 may be mounted at the endoscope system control device 5 and the observation monitor 6, respectively, so that an image signal is sent from the endoscope system control device 5 to the observation monitor 6 and an endoscope image corresponding to the image signal is displayed on its display surface.

As will be described later, to the endoscope system control device 5, image data picked up by the CCD 25 from the AWS unit 4 side and image data of the insertion portion shape (UPD image) of the endoscope 3 detected using the UPD coil unit 8 are sent, and thus, the endoscope system control device 5 sends an image signal corresponding to these image data to the observation monitor 6 so that the UPD image can be also displayed with the endoscope image on the display surface.

The observation monitor 6 comprises a monitor of a high definition TV (HDTV) so that plural types of images can be displayed on the display surface at the same time in this way.

In this embodiment, a recess portion for housing as the housing portion 2b of the scope tray 39 is formed at one end in the longitudinal direction in the laying table 2a of the inspection bed 2 and a position below it so that an upper part of a trolley 38 for carrying a tray can be freely slidably housed in this housing portion 2b. This tray-carrying trolley 38 is loaded with the scope tray 39, which is a tray for an endoscope, and carries the scope tray 39 into the housing portion 2b and out of the housing portion 2b. At an upper part of this tray-carrying trolley 38, a tray loaded portion 38a is provided to be loaded with the scope tray 39 where the endoscope 3 shown in Fig. 9 is housed. Thus, the housing portion 2b of the scope tray 39 is formed by the housing-recess portion 2b and the tray loaded portion 38a. Also, the scope tray 39 can be removed from the tray loaded portion 38a, can be removed from the housing portion 2b and moreover, it can be carried together with the trolley 38 loaded with the scope tray 39 on the tray loaded portion 38a.

Then, the scope tray 39 in which the disinfected or sterilized endoscope 3 is housed can be carried by the tray-carrying trolley 38 and can be housed in the housing-recess portion 2b of the inspection bed 2. The operator withdraws the endoscope 3 from the scope tray 39 to use it for an endoscope inspection and houses it in the scope tray 39 again after the endoscope inspection is finished. After that, by carrying the scope tray 39, in which the endoscope 3 often use is housed, using the tray-carrying trolley 38, disinfection or sterilization can be performed smoothly.

Particularly, as shown by a two-dotted chain line in Fig. 4, a patient lies down with his lower body faced toward the UPD coil unit 8 side for a colon inspection, for example. Therefore, after completion of the inspection, when the insertion portion is pulled out of the body cavity of the patient on the UPD coil unit 8, the operator can immediately place the endoscope in the scope tray 39 in the vicinity of the UPD coil unit 8 and easily put away the endoscope after use by pushing the tray-carrying trolley 38 which is loaded with the scope tray 39.

Also, as shown in Fig. 4, a scope connector 40 is provided at the AWS unit 4, for example. And to this scope connector 40, as shown in Fig. 8, a scope connector 41 of the endoscope 3 is freely detachably connected.

In this case, more specific shapes of appearance of the scope connector 40 on the AWS unit 4 side are shown in Figs. 5, 6A and 6B. Also, Figs. 7A to 7E show the structure of an AWS adapter 42 which is freely detachably mounted to the scope connector 40 of the AWS unit 4, and Fig. 8 shows the internal structures of the scope connector 40 on the AWS unit 4 side and the scope connector 41 on the endoscope 3 side in the connected state.

Actually, as shown in Fig. 6B, an AWS adapter mounting portion 40a in the recess shape is provided on the front face of the AWS unit 4, and the scope connector 40 is formed by mounting the AWS adapter (pipeline connection adapter) 42 shown in Figs. 7A to 7E at this AWS adapter mounting portion 40a, and the scope connector 41 of the endoscope 3 is connected to this scope connector 40.

At the AWS adapter mounting portion 40a, an electric connector 43 for scope connection, an air-supply connector 44 and a pinch valve 45 are provided, and an inner end face of the AWS adapter 42 is freely detachably mounted at this AWS adapter mounting portion 40a and from its outer end face side, the scope connector 41 of the endoscope 3 is connected.

Details of this AWS adapter 42 are shown in Figs. 7A to 7E. Fig. 7A is a front view of the AWS adapter 42, Fig. 7B is a left side view of the AWS adapter 42, Fig. 7C is a right side view of the AWS adapter 42, Fig. 7D is a sectional view along A-A' line in Fig. 7A, and Fig. 7E is a sectional view along B-B' line in Fig. 7A.

To this AWS adapter 42, the scope connector 41 is inserted at the recess portion 42a on its front face, and in this case, the electric connector portion in the scope connector 41 is inserted into a through hole 42b provided in this recess portion and connected to the electric connector 43 for scope connection provided at the AWS unit 4 disposed in this through hole 42b.

Also, an air/water supply connector 42c and a suction connector 42d are arranged at a lower part of this through hole 42b, to which an air/water supply base 63 and a suction base 64 in the scope connector 41 (See Figs. 8 and 9) are connected, respectively.

On the base end face side of the AWS adapter 42, a recess portion 42f housing a pinch valve 45 projecting from the AWS adapter mounting portion 40a is provided.

As shown in Fig. 7E, in the air/water supply connector 42c provided at the AWS adapter 42, an internal pipeline communicating with this is branched to an air-supply base 42e to be connected to the air-supply connector 44 of the AWS unit 4 and a water supply base 46 projecting sideward. Also, in the suction connector 42d, a pipeline communicating with this is bent sideward to become a suction base 47 projecting to a side face and branched upward in the middle, for example, to become a relief pipeline 47a. This relief pipeline 47a is passed through the inside of the pinch valve 45 in the middle and its upper end is opened.

When a suction pump, not shown, constituting suction means, is set to an all-the-time operating state, this relief pipeline 47a is usually set to a release state by the pinch valve 45, and the pinch valve 45 is driven when a suction operation is carried out. And the relief pipeline 47a is closed by this pinch valve 45, whereby release is stopped and the suction operation is carried out.

The water supply base 46 and the suction base 47 are, as shown in Fig. 5 and the like, connected to a water feed tank 48 and an aspirator (with a suction tank 49b interposed in the middle through a suction tube 49a), respectively. The feed water tank 48 is connected to a connector 50 for a feed water tank. An operation panel 4a is provided at an upper part side of the scope connector 40 on the front face of the AWS unit 4.

Next, a specific configuration of the endoscope 3 of the embodiment of the present invention will be described referring to Fig. 9.

As its outline has been described in Fig. 3, the endoscope 3 of this embodiment comprises the endoscope main body 18 having the flexible insertion portion 21 and the operation portion 22 provided at its back end and the disposable type (abbreviated as dispo type) tube unit 19 to which a comprehensive connector portion 52 of its proximal end is detachably connected at a connector portion 51 (for tube unit connection) provided in the vicinity of the base end (front end) of the operation portion 22 in this endoscope main body 18, and at the terminal end of this tube unit 19, the above-mentioned scope connector 41 to be freely detachably connected to the AWS unit 4 is provided.

The insertion portion 21 comprises the rigid tip portion 24 provided at a distal end of this insertion portion 21, the curved portion 27 which is provided at the back end of the tip portion 24 and is capable of being curved, and an elongated flexible portion (hose portion) 53 from the back end of this curved portion 27 to the operation portion 22, and actuators 54A, 54B for rigidity changing also referred to as electroconductive polymer artificial muscle (abbreviated as EPAM) which is expanded/contracted by application of voltage and can change rigidity are provided at plural locations in the middle of this flexible portion 53, specifically at two locations.

Inside an illumination window provided at the tip portion 24 of the insertion portion 21, a light emitting diode (hereinafter abbreviated as LED) 56, for example, is provided as illumination means, and the illumination light from this LED 56 is emitted forward through an illumination lens integrally provided at this LED 56 so as to illuminate a subject such as an affected portion. This LED 56 may be an LED emitting white light or may be constructed using an LED for R, an LED for G and an LED for B emitting light of each wavelength region of red (R), green (G) and blue (B). A light emitting device constituting the illumination means is not limited to the LED 56, but it may be LD (laser diode) or the like.

Also, at an observation window provided adjacent to this illumination window, an objective lens, not shown, is provided, and at its image forming position, the CCD 25 incorporating a gain changing function is provided so as to form image pickup means for capturing an image of the subject. Since the CCD 25 in this embodiment incorporates the gain changing function in the CCD device itself and can easily change the gain of a CCD output signal to about several hundred times by the gain changing function, a bright image with less S/N drop can be obtained even under the illumination light by the LED 56. Also, since the LED 56 has more favorable light emitting efficiency than that of a lamp, temperature rise around the LED 56 can be restrained.

Having one end connected to the LED 56 and the CCD 25, respectively, the other end of the signal line inserted into the insertion portion 21 is connected to a control circuit 57 provided inside the operation portion 22, for example, for performing centralized control processing (intensive control processing).

Also, in the insertion portion 21, a plurality of UPD coils 58 are provided with a predetermined interval along its longitudinal direction, and a signal line connected to each of the UPD coils 58 is connected to the control circuit 57 through an UPD coil driving unit 59 provided in the operation portion 22.

Moreover, at four locations in the circumferential direction inside the outer skin in the curved portion 27, an actuator 27a for angle with being formed by arranging EPAM in the longitudinal direction, is arranged. And the actuators 27a for angle and the actuators 54A, 54B for rigidity changing are connected to the control circuit 57 through the signal lines, respectively.

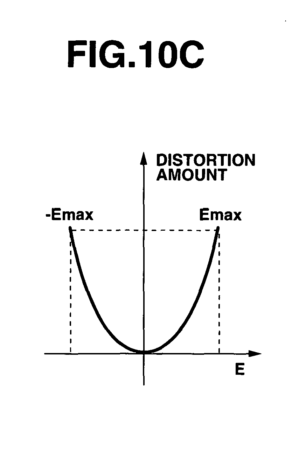

The EPAM used in the angle actuators 27a and the rigidity changing actuators 54A, 54B can be contracted in the thickness direction and expanded in the longitudinal direction as shown in Fig. 10B by mounting electrodes on both faces of the EPAM in the plate shape as shown in Fig. 10A, for example, and applying voltage. With regard to this EPAM, its distortion amount can be changed in proportion to approximately square of an electric field strength E generated by the applied voltage, for example, as shown in Fig. 10C.

When it is used as the angle actuator 27a, it may be formed in the wire shape or the like to expand one side and to contract the opposite side so that the curved portion 27 can be curved similarly to the function by a usual wire. Also, the rigidity can be changed by this expansion or contraction, and the rigidity changing actuators 54A, 54B use that function to make the rigidity of the portion changed.

Moreover, in the insertion portion 21, an air/water supply pipeline 60a and a suction pipeline 61a are inserted, and the back end constitutes a pipeline connector portion 51 a opened at the connector portion 51. And to this pipeline connector portion 51a, a tube connector 52a in the comprehensive connector portion 52 at the proximal end of the tube unit 19 is freely detachably connected.

And the air/water supply pipeline 60a is connected to an air/water supply pipeline 60b inserted into the tube unit 19, and the suction pipeline 61 a is connected to a suction pipeline 61 b inserted into the tube unit 19 and branched in the tube connector 52a and opened outward so as to communicate with a treatment instrument insertion port capable of insertion of a treatment instrument such as forceps (hereinafter abbreviated as forceps port) 62. This forceps port 62 is blocked by a forceps plug 62a when not in use.

The back ends on the proximal side of these air/water supply pipeline 60b and the suction pipeline 61b constitute the air/water supply base 63 and the suction base 64 in the scope connector 41.

The air/water supply base 63 and the suction base 64 are connected to the air/water supply connector 42c and the suction connector 42d of the AWS adapter 42 shown in Figs. 6A to 7E, respectively. And the air/water supply connector 42c is branched into the air supply pipeline and the water supply pipeline inside this AWS adapter 42 as shown in Figs. 7A to 7E. As shown in Fig. 8, the air supply pipeline is connected to a pump 65 for air/water supply inside the AWS unit 4 with an electromagnetic valve B1 interposed, while the water supply pipeline is connected to the water feed tank 48. This water feed tank 48 is also connected to the pump 65 for air/water supply with an electromagnetic valve B2 interposed in the middle. The pump 65 for air/water supply, the electromagnetic valves B1 and B2 are connected to an AWS control unit 66 by a control line (driving line), and opening/closing thereof is controlled by this AWS control unit 66 so that air supply and water feed can be carried out. The AWS control unit 66 also performs suction operation control by control of opening/closing of the pinch valve 45.

Also, as shown in Fig. 9, a gripping portion 68 to be gripped by an operator is provided at the operation portion 22 of the endoscope main body 18, and in the periphery of this gripping portion, there are provided three scope switches SW1, SW2, SW3, for example, which perform remote control operation such as release, freeze and the like (hereinafter abbreviated as remote control operation), along the longitudinal axis of the operation portion 22 and they are connected to the control circuit 57, respectively.

Moreover, on an inclined surface portion Sa formed as an upper surface with inclination opposite to the position where these scope switches SW1, SW2, SW3 are provided in the operation portion 22, a track ball 69 in the waterproof structure is provided for angle operation (curving operation) or for setting other remote control operations and the like by switching at a position capable of operation by a hand gripping the gripping portion 68.

A view on arrow C in Fig. 9 is shown in Fig. 11. As shown in Fig. 11, on both sides of the track ball 69 in this inclined surface portion Sa, two scope switches SW4, SW5 are provided at positions in the horizontal direction in symmetry with respect to the longitudinal direction of the operation portion 22. To the scope switches SW4, SW5, functions of an air/water supply switch and a suction switch are usually allocated.

A view when the operation portion 22 of the endoscope 3 is seen along the arrow C direction in Fig. 9 is set as a front, the track ball 69 is located on the center line in the longitudinal direction with respect to the longitudinal direction of the operation portion 22 or the insertion portion 21, and the two scope switches SW4, SW5 are arranged in symmetry and the scope switches SW1, SW2, SW3 are arranged along this center line on the rear face side.

In this way, since the various operating means such as the track ball 69 are provided in symmetry with respect to the center axis in the longitudinal direction of the operation portion 22, when the operator grips the gripping portion 68 of the operation portion 22 for operation, favorable operating performance can be ensured similarly in both operations of gripping by the left hand and of gripping by the right hand.

This track ball 69 and the scope switches SW4, SW5 are connected to the control circuit 57. The track ball 69 and the scope switches SW1 to SW5 correspond to the angle/remote control operator 28 in Fig. 3.

Also, a power line 71 a and a signal line 71 b extended from this control circuit 57 are electrically connected to a power line 73a and a signal line 73b inserted into the tube unit 19 through contactless transfer portions 72a, 72b formed in the connector portion 51 and the comprehensive connector portion 52 in the contactless manner (For details, see Fig. 12). The power line 73a and the signal line 73b are connected to an electric connector 74 provided with a power and signal contact in the scope connector 41. The connector portion 51 side in the contactless transfer portions 72a, 72b is referred to as a contactless transfer unit 51b, for example.

And with this scope connector 41 being connected to the AWS unit 4 by the user, the power line 73a is connected to a power supply unit 75 through the electric connector 43 of the AWS unit 4 as shown in Fig. 8, while the signal line 73b is connected to an UPD unit 76 and the sending/receiving unit 77 through the power supply unit 75 and to the AWS control unit 66. The sending/receiving unit 77 is connected to an antenna for sending/receiving an electric wave by wireless.

Fig. 12 shows a configuration of a contactless connection portion constituted of the contactless transfer portions 72a and 72b in the connector portions 51 and 52.

Alternating-current power supplied from the power supply unit 75 by the power line 73a inserted into the tube unit 19 is supplied to a coil C1a on the primary side housed in an armor case of the connector portion 52 and forming the contactless transfer portion 72a.

Inside the armor case of the connector portion 51, a coil C1b on the secondary side is arranged, and the above primary side coil C1a and the secondary side coil C1b are brought close to each other and form a transducer T1 electromagnetically coupled in the state with least flux leakage.

And by this electromagnetic coupling, the alternating-current power supplied to this coil C1a is transmitted to the secondary side coil C1b efficiently. This coil C1b is connected to a power circuit 78 in the control circuit 57 and generates direct power by the power circuit 78 required on the control circuit 57.

The power circuit 78 converts the direct-current voltage rectified through a diode D for rectification and a smoothing capacitor to a direct-current voltage required for the operation of the control circuit 57 by an IC 79 for 3-terminal power supply and the smoothing capacitor, for example, and supplies it to the control circuit 57.

Also, the signal line 71b connected to the control circuit 57 (and forming common signal transfer means) is connected to a coil C2a constituting the contactless transfer portion 72b, and a coil C2b close to and opposite to this coil C2a is connected to the signal line 73b inserted into the tube unit 19. That is, almost similarly to the case of transducer T1, the contactless transfer portion 72b is constituted of the transducer T2 electromagnetically coupled by the coils C2a and C2b.

A signal is transmitted from the signal line 71b side to the signal line 73b side through the electromagnetically coupled coils C2a and C2b, and the signal is also transmitted in the opposite direction.

In this embodiment, as its internal configuration described in Fig. 13, by configuring so that the various operating means and image capturing means and the like are controlled or managed by the control circuit 57 in the centralized manner, the number of electric signal lines inserted in the tube unit 19 can be reduced. Also, even if the function provided at the endoscope 3 is changed, the signal line 73b in the tube unit 19 can be used as it is without a change. That is, the signal line 73b forms the common signal transfer means for transferring various signals in common.

As shown in Fig. 12, when magnets M1 and M2 are arranged so that different magnetic poles are opposed to each other adjacent to the transducer T2 and the comprehensive connector portion 52 is connected to the connector portion 51, for example, the coils C1a and C1b as well as the coils C2a and C2b can be freely detachably mounted in a facing manner in proximity. In place of the magnets M 1 and M2, an irregular portion fitting with both the connector portions 51, 52 may be provided for positioning.

The endoscope 3 of this embodiment is characterized in that the endoscope main body 18 is freely detachably connected to the tube unit 19 in the contactless manner in this way.

Fig. 13 shows a configuration of the electric system of the control circuit 57 and the like arranged in the operation portion 22 of the endoscope main body 18 and the major components arranged at each portion of the insertion portion 21.

At the tip portion 24 of the insertion portion 21 shown at a lower part in the left in Fig. 13, the CCD 25 and the LED 56 are arranged, and at the curved portion 27 depicted above them in the figure, the angle actuator (specifically, EPAM in this embodiment) 27a and an encoder 27c are arranged.

Also, at the flexible portion 53, the rigidity changing actuator 54 and an encoder 54c (specifically, they are rigidity changing actuators 54A, 54B by EPAM in this embodiment, but they are simplified and represented by one) are arranged respectively. Also, at this flexible portion 53, the UPD coils 58 are arranged.

Moreover, on the surface of the operation portion 22 described above, the flexible portion 53 of the insertion portion 21, the track ball 69, the air/water supply SW (SW4), the suction SW (SW5) and the scope SW (SW1 to 3) are arranged. The track ball 69 is used for angle operation and selection setting of other functions and the like as will be described later.

Those shown in the left of Fig. 13 are connected to the control circuit 57 provided in the operation portion 22 shown in their right through the signal line (the UPD coil driving unit 59 is in the operation portion 22), and the control circuit 57 performs driving control of those functions and signal processing and the like.

The control circuit 57 has a state management portion 81 comprising a CPU and the like for managing a control state, and this state management portion 81 is connected to a state maintenance memory 82 for maintaining (storing) the state of each portion. This state maintenance memory 82 has a program storing memory 82a as control information storing means, and by rewriting program data as control information stored in this program storing memory 82a, even if the components shown in Fig. 13 are changed, the CPU constituting the state management portion 81 can perform control (management) corresponding to the changed configuration.

Also, this state maintenance memory 82 or at least the program maintaining memory 82a is constituted of a flash memory which is non-volatile and can be electrically rewritten, for example, or EEPROM or the like so that the program data can be easily changed through the state management portion 81.

The program data can be changed by sending a command to change the program data to the state management portion 81 through a signal line 71 b, for example, that is, through a wired sending/receiving unit 83 below and by sending program data for rewrite after the command from the AWS unit 4 side is issued. Also, upgrade and so on can be easily realized through the signal line 71b.

Also, the state maintaining memory 82 may maintain model information specific to each endoscope 3 or individual information corresponding to a use situation as follows by writing so that the information can be effectively used. Specifically, the state maintaining memory 82 maintains the model information of the endoscope 3 (type of the CCD 25, information such as the insertion portion length, for example), for example, as well as the individual information of each endoscope 3 which is different depending on the use situation of the endoscope inspection or the like (information such as use time (aggregated or accumulated use time of endoscope inspection), the number of washing times, adjustment value, maintenance history or the like, for example), and this information is used for determination of system operation or information provision to the user.

This information can be also edited by the endoscope system control device 5 or from the outside such as a washing device, not shown.

In this way, by sharing and using the state maintaining memory 82 also for the function of the conventional scope ID, the information to be given to the scope ID, that is, data can be effectively utilized.

Also, since this state maintaining memory 82 is provided, there is no need to provide a separate scope ID. The function can be made more sophisticated than that of the existing scope ID, and appropriate setting, adjustment, management, processing and the like in more detail can be realized.

Moreover, this state management portion 81 is connected to the wired-type sending/receiving unit 83 for performing wired communication with the AWS unit 4 in this embodiment (this sending/receiving unit 83 corresponds to Fig. 2B, and the components are shown with the reference numerals in Fig. 2B. However, the electric connector 15 constitutes the contactless transfer portions 72a, 72b in the operation portion 22 and the electric connector 74 at the end of the tube unit 19).

Furthermore, this state management portion 81 controls an LED driving portion 85 controlled through an illumination control portion 84 controlling illumination. This LED driving portion 85 applies an LED driving signal for having the LED 56 constituting the illumination means emit to the LED 56.

By light emitting of this LED 56, an image of the subject such as an illuminated affected area is formed by an objective lens, not shown, mounted at the observation window on an image pickup surface of the CCD 25 arranged at its image forming position and photoelectrically converted by this CCD 25.

This CCD 25 outputs as an image pickup signal a signal charge accumulated by photoelectric conversion which is performed by application of a CCD driving signal from a CCD driving portion 86 controlled by the state management portion 81. This image pickup signal is converted by an A/D converter (hereinafter abbreviated as ADC) 87 from an analog signal to a digital signal and then, inputted to the state management portion 81, and the digital signal (image data) is stored in an image memory 88. The image data of this image memory 88 is sent to a data sending portion 12' of the sending/receiving unit 83.

And it is transferred from the electric connector 15 (the contactless transfer unit 51b in this embodiment) to the AWS unit 4 side through the signal line 73b in the tube unit 19. Moreover, it is transferred to the endoscope system control device 5 from the AWS unit 4 in the wireless manner.

An output signal of the above ADC 87 is sent to a brightness detection portion 89, and information on brightness of the image detected by the brightness detection portion 89 is sent to the state management portion 81. The state management portion 81 performs light control through the illumination control portion 84 on the basis of this information so that the illumination light amount by the LED 56 becomes appropriate brightness.

Also, the state management portion 81 controls an actuator driving portion 92 through an angle control portion 91 for management to drive the angle actuator (EPAM) 27a by this actuator driving portion 92. The driving amount of this angle actuator (EPAM) 27a is detected by the encoder 27c and controlled so that the driving amount matches a value corresponding to an instructed value.

Moreover, the state management portion 81 controls an actuator driving portion 94 through a rigidity changing control portion 93 for management to drive the rigidity changing actuator 54 by this actuator driving portion 94. The driving amount of this rigidity changing actuator 54 is detected by the encoder 54c and controlled so that the driving amount becomes a value corresponding to an instructed value.

Moreover, to this state management portion 81, an operation signal corresponding to the operation amount from the track ball 69 and the like provided at the operation portion 22 is inputted through a track ball displacement detection portion 95.

Furthermore, switch pressing operation to turn on or the like by the air/water supply SW, suction SW, scope SW is detected by a switch pressing detection portion 96 and the detected information is inputted to the state management portion 81. EPAM has a characteristic to generate an electromotive force caused by deformation by an external force, and the EPAM arranged on the side opposite to the EPAM to be driven may be used as an encoder.

Also, the control circuit 57 has a power transfer receiving portion 97 and a power generation portion 98. The power transfer receiving portion 97 specifically constitutes the contactless transfer portion 72a at the operation portion 22. And an alternating-current power transferred to the power generation portion 98 is converted to a direct current at this power generation portion 98. This power generation portion 98 corresponds to the power circuit 78 in Fig. 11. The direct current power generated by the power generation portion 98 supplies electric power required for operation of each portion inside the control circuit 57.

Fig. 14 shows internal configurations of the sending/receiving unit 101 and an image processing unit 116, shown in Fig. 8, of the endoscope system control device 5.

This endoscope system control device 5 has the sending/receiving unit 101 in the wireless method, for example. Data such as an image signal sent by wireless from the AWS unit 4 is taken in by the antenna portion 13, sent to the data receiving portion 14 and amplified and then, demodulated. The operation of this data receiving portion 14 is controlled by the data communication control portion 11, and the received data is sequentially accumulated in a buffer memory 102.

The image data of this buffer memory 102 is sent to an image processing portion 103 which performs image data processing. To this image processing portion 103, character information from a character generation portion 105 generating character information by key input of a keyboard 104 is inputted in addition to the image data from the buffer memory 102, and the image processing portion 103 can superimpose and the like the character information on the image data.

The image processing portion 103 sends the inputted image data and the like to an image memory control portion 106 and temporarily stores the image data and the like in an image memory 107 through this image memory control portion 106 and records it in a recording media 158.

Also, the image memory control portion 106 reads out the image data temporarily stored in the image memory 107 and sends it to a digital encoder 108, and the digital encoder 108 encodes the image data in a predetermined image method and outputs it to a D/A converter (hereinafter abbreviated as DAC) 109. This DAC 109 converts a digital image signal to an analog image signal. This analog image signal is further outputted from an image output end to the observation monitor 6 through a line driver 110, and an image corresponding to the image signal is displayed on the observation monitor 6.

Moreover, the image data temporarily stored in the image memory 107 is read out and inputted also to a DV data generation portion 111, and DV data is generated by this DV data generation portion 111 and the DV data is outputted from a DV data output end.

Furthermore, in this endoscope system control device 5, an image input end and a DV data input end are provided, and an image signal inputted from the image input terminal and converted to a digital signal through a line receiver 112, and an ADC 113 is demodulated by a digital decoder 114 and inputted to the image memory control portion 106.

Also, as for the DV data inputted to the DV data input end, the image data is extracted (decoded) by an image data extraction portion 115 and inputted to the image memory control portion 106.

The image memory control portion 106 also temporarily stores in the image memory 107 the image signal (image data) inputted from the image input end or the DV data input end, records it in the recording media 158 or outputs it from the image output end to the observation monitor 6.

In this embodiment, image data picked up by the CCD 25 of the endoscope 3 and UPD image data generated by the UPD unit 76 are inputted from the AWS unit 4 side to the endoscope system control device 5 by wireless, and the endoscope system control device 5 converts the image data into a predetermined image signal and outputs it to the observation monitor 6. The endoscope system control device 5 may receive an UPD coil position data instead of the UPD image data to generate the UPD image data in the image processing portion 103.

Fig. 15 shows the internal configuration of the AWS unit 4.

The image data and operation data of the switch and the like inputted to the electric connector 43 for scope from the control circuit 57 of the endoscope 3 is outputted to the data communication control portion 11 of the sending/receiving unit 77 and sent from the antenna portion 13 to the antenna portion 13 of the endoscope system control device 5 together with the UPD image data sent from the UPD unit 76.

On the other hand, AWS related information such as operation of the air/water supply switch and the suction switch provided at the operation portion 22 of the endoscope 3 is also sent to an air/water supply control portion 122, and this air/water supply control portion 122 controls operation of the pump 65 and an electromagnetic valve unit 124 in correspondence to the operated information. To the electromagnetic valve unit 124, air/water supply tubes 60b, 61 b are connected through the AWS adapter 42. Also, to the electromagnetic valve unit 124 and the AWS adapter 42, the water feed tank 48 is connected, and to the AWS adapter 42, the suction tank 49b is connected.

Moreover, to the AWS unit 4, commercial power source is supplied, and this commercial power source is sent to a power source transfer output portion 127 through an insulating transducer 126. This power transfer output portion 127 supplies alternating-current power, which is insulated from the commercial power source, from the electric connector 43 to the power line 73a of the endoscope 3 connected to this electric connector 43.

A power transfer output of the power transfer output portion 127 is controlled by a power transfer control portion 128 connected to the data communication control portion 11.

In the endoscope system 1 provided with this embodiment, the observation monitor 6 displays various images as shown in Fig. 16A, for example, when it is powered on. In this case, there are provided an information display area Rj for displaying patient information and the like, a display area Ri for an endoscope image, a display area Ru for an UPD image, a display area Rf for a freeze image and a display area Ra for an angle shape as well as a menu display area Rm, and a menu is displayed in the menu display area Rm. The angle-shape display area Ra detects an angle operation amount of the angle actuator 27a by the encoder 27c and displays the angle shape in that case.

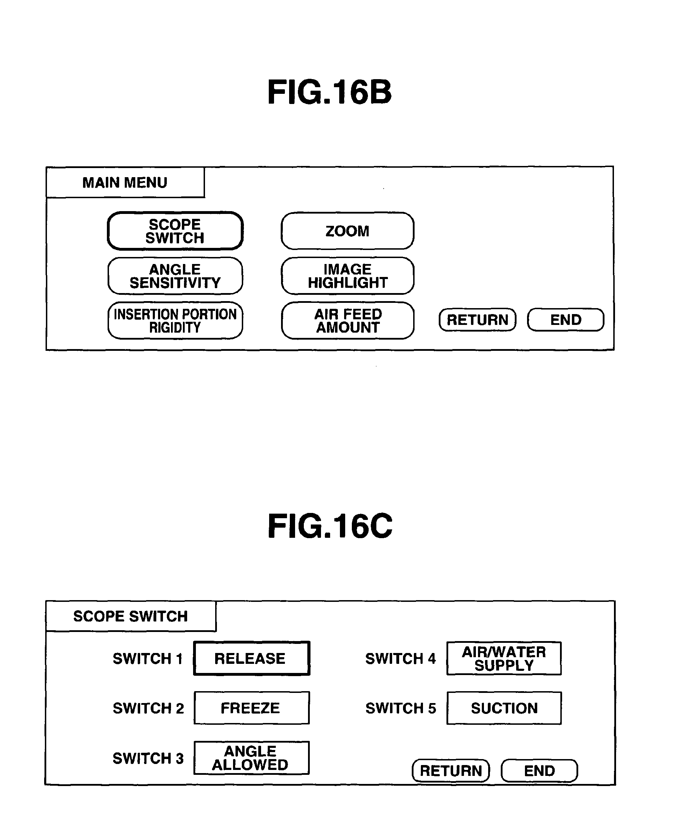

As a menu displayed in the menu display area Rm, a main menu shown in Fig. 16B is displayed. This main menu displays items of "Scope switch", "Angle Sensitivity", "Insertion portion rigidity", "Zoom", "Image highlight", "Air supply amount" and "Return" to instruct operation to return to the previous menu screen and "End" to instruct operation to end the menu.

When the user moves/selects the selection frame to the item of the scope switch by operation of the track ball 69 and the like, the frame of the scope switch item is displayed in bold to indicate that it is selected, and further by performing determination operation by pressing the track ball 69, the function to be allocated to the five scope switches SW1 to SW5 can be selected/set as shown in Fig. 16C.

Next, action of the endoscope system 1 in this configuration will be described.

As a preparation for conducting the endoscope inspection, the comprehensive connector portion 52 on the dispo type tube unit 19 side is connected to the connector portion 51 of the operation portion 22 of the endoscope main body 18. In this case, the transducers T1, T2 constituting the contactless transfer portions 72a, 72b are electromagnetically connected in the state insulated to each other and waterproof. By this connection, preparation of the endoscope 3 is completed.

Next, the scope connector 41 of the tube unit 19 is connected to the connector 43 of the AWS unit 4. This portion completes one-touch connection of various pipelines, power lines, signal line and optical connection in one connection operation. It is not necessary to perform connection of various pipelines or connection of electric connector every time as in the conventional endoscope system.

Also, the user connects the AWS unit 4 to the UPD coil unit 8 and connects the endoscope system control device 5 to the observation monitor 6. Moreover, by connecting the endoscope system control device 5 to the image recording unit 7 and the like, as needed, setup of the endoscope system 1 is completed.

Next, the AWS unit 4 and the endoscope system control device 5 are powered on. Then, each portion in the AWS unit 4 is brought into the operating state and the power supply unit 75 can supply power to the endoscope 3 side through the power line 73a and the like.

The operation at start of the AWS unit 4 and the endoscope 3 in this case will be described referring to Figs. 17 and 18.

The power transfer control portion 128 inside the power supply unit 75 of the AWS unit 4 shown in Fig. 15 brings the state of the power transfer output portion 127 to power-supply stop, that is, the power supply is turned OFF in the first Step S1 as shown in Fig. 17 when the start processing is started.

After that, at step S2, the monitor timer is turned ON and then, as shown in Step S3, the state of the power transfer output portion 127 is brought into the state of power supply, that is, the power supply is turned ON. When the power transfer output portion 127 starts power supply, the alternating-current power is supplied through the power line 73a in the tube unit 19 and further through the contactless transfer portion 72a to the power generation portion 98 in the control circuit 57 of the operation portion 22.

After that, as shown in Step S4, the power transfer control portion 128 is brought into the state of waiting for receiving of a start-up message from the endoscope 3 through the signal line 73b in the tube unit 19. And when the power transfer control portion 128 does not receive the start-up message, as shown in Step S5, determination is made if the monitor timer has expired or not, and if it is not, the routine goes back to Step S4, while if it has expired, the routine returns to the first Step S1.

On the other hand, at step S4, if the start-up message is received before expiration, the power transfer control portion 128 turns OFF time measurement by the monitor timer as shown in Step S6. And as shown in Step S7, a continue message is issued and this start-up processing is finished.

On the other hand, since the alternating-current power is supplied to the power generation portion 98, power required for operation in the control circuit 57 is supplied to the control circuit 57 of the endoscope 3, and the start-up processing is started. And the state management portion 81 shown in Fig. 13 waits for stabilization of the power voltage of the power generation portion 98 at the first Step S11.

And when the power voltage is stabilized, at the next Step S12, the state management portion 81 performs system initialization of each portion in the control circuit 57. After this system initialization, as shown in Step S 13, the state management portion 81 sends the start message through the sending/receiving unit 83 and further through the signal line 73b in the tube unit 19 to the power transfer control portion 128.

After sending this start-up message, as shown in Step S14, the state management portion 81 is brought into the state waiting for the continue message from the power transfer control portion 128, and when the continue message is received, the start-up processing is ended. On the other hand, if the continue message is not received, as shown in Step S 15, if the condition for retry end (condition of the number of retry times set in advance, for example) is not reached, the state management portion 81 returns to step S 13, where the start-up message is issued again and if the retry end condition is met, the routine is ended in error.

When the above start-up processing is ended normally, image pickup by the CCD 25 is started, and the user can perform air/water supply, suction, angle operation, rigidity changing operation and the like by the operating means of the operation portion 22.

Typical processing operations relating to these will be described referring to Figs. 19 to 22. Fig. 19 shows an operation content of the image capturing control processing.

As shown in Fig. 19, when the image pickup processing is started, as shown in Step S21, the endoscope 3 obtains image pickup data. Specifically, under management (control) of the state management portion 81, the LED 56 emits light, the CCD driving portion 86 starts operation to drive the CCD 25, and an image signal picked up by the CCD 25 is converted by the ADC 87 to the digital signal (image pickup data). The image pickup data (image data) is sequentially stored in the image memory 88, and image pickup data is obtained.

The obtained image data is sequentially sent as shown in Step S22. The image data read out of the image memory 88 is sent through wire from the sending/receiving unit 83 to the AWS unit 4 and sent from the sending/receiving unit 77 of this AWS unit 4 to the endoscope system control device 5 wirelessly, converted to the image signal inside the endoscope system control device 5 and displayed on the observation monitor 6.

Also, the image pickup data of the ADC 87 is inputted to the brightness detection portion 89. As shown in Step S23, this brightness detection portion 89 detects brightness of the image pickup data by calculating an average value in an proper time of the luminance data of the image pickup data.

The detection data of this brightness detection portion 89 is inputted to the state management portion 81, for example, to determine if it is specified brightness or not (Step S24). And if it is specified brightness, the image capturing processing is ended and moved to the next image capturing processing.

On the other hand, at Step S24, if it is determined that the brightness is not specified, as shown in Step S25, the state management portion 81 sends an instruction signal (control signal) for illumination light adjustment to the illumination control portion 84, and the illumination control portion 84 adjusts an illumination light amount. For example, the illumination control portion 84 adjusts the illumination light amount by increasing or decreasing a driving current which has the LED 56 emit light. The illumination control portion 84 returns this adjustment result to the state management portion 81.

Therefore, the state management portion 81 determines from the information of the adjustment result if it is within a brightness adjustment range which can be realized by the illumination control portion 84. If brightness adjustment by the illumination control portion 84 is possible, this image pickup processing control is finished without processing at Step S27. On the other hand, if it is out of the brightness adjustment range by the illumination control portion 84, as shown in Step S27, the state management portion 81 outputs a signal for CCD gain control to the CCD driving portion 86 and adjusts the brightness of the image pickup data by adjusting the gain of the CCD 25. And this image capturing processing is finished.

Next, air/water supply processing in Fig. 20 will be described. As shown in Fig. 11, functions of the air/water supply switch and the suction switch are usually allocated to both sides of the track ball 69 in the operation portion 22.

When the air/water supply processing is started, as shown in Step S31 in Fig. 20, the state management portion 81 of the control circuit 57 obtains state data of the air/water supply switch.

Operation of the air/water supply switch is detected by the switch pressing detection portion 96 shown in Fig. 13, and when the information of the detection result is inputted, the state management portion 81 obtains the state data of the air/water supply switch.

And as shown in Step S32, the state management portion 81 determines change in the state of the air/water supply switch. If it is determined at Step S32 that the state of the air/water supply switch is changed, the state management portion 81 sends air/water supply control data corresponding to the instruction of the air/water supply switch operated by the user to the AWS unit 4 through the sending/receiving unit 83 as shown in Step S33.

The air/water supply control portion 122 at the AWS unit 4 performs control operation of the pump 65 and the electromagnetic valve unit 124 in correspondence to this air/water supply control data. And this air/water supply processing operation is finished. On the other hand, at Step S32, if it is determined that there is no state change in the air/water supply switch, this air/water supply processing operation is finished without processing at Step S33. Since suction processing is substantially similar to the air/water supply processing, its description is omitted.

Next, processing of the angle operation control will be described referring to Fig. 21. When the angle control processing is started, as shown in Step S41, the state management portion 81 determines if the angle control is effective or not.

In this embodiment, the state management portion 81 determines if the angle control is effective or not as shown in Step S41 on the basis of whether the track ball 69 is pressed down or not. Specifically, the state management portion 81 can detect a displacement operation and a pressing operation of the track ball 69 by output of the track ball displacement detection portion 95. If the track ball 69 is pressed, the angle control is turned OFF.

The state management portion 81 determines if the angle control is effective or not by the output of the track ball displacement detection portion 95.

And if it is determined that the angle control is not effective, the routine moves to Step S45, where the previous command value is maintained. On the other hand, if it is determined that the angle control is effective, the routine goes on to the next Step S42, and the state management portion 81 obtains the state data by operation of the track ball 69. And at the next Step S43, the state management portion 81 determines if there is state change or not by output of the track ball displacement detection portion 95.

In this case, if the state management portion 81 determines that there is no state change, the routine goes to Step S45, while if it determines that there is a state change, a command value corresponding to the rotating direction and rotating amount of the track ball 69 are calculated at the next Step S44.

After the processing of Step S44 or S45, the state management portion 81 sends the command value to the actuator driving portion 92 through the angle control portion 91 as shown in Step S46 and performs servo processing of the actuator for angle.

That is, the actuator driving portion 92 drives the actuator for angle so that an angle state (curved angle) corresponding to the command value can be obtained on the basis of the command value. At that time, the angle state of the actuator for angle is detected by the encoder, and the actuator driving portion 92 drives the angle actuator so that a value detected by this encoder matches the command value. The angle control processing is finished in this way.

In Fig. 21, a processing operation when a touch sensor is provided at the servo processing of step S46 is also shown (Steps S47 and S48). The processing of steps S47 and S48 is as follows.

If the touch sensor is provided, when the state management portion 81 performs the angle operation control for the angle operation, the state management portion 81 takes in a detection result obtained by a touch sensor 142 through a touch sensor detection portion 147 as shown in Step S47 during start-up of the servo processing by Step S46 and detects (determines) if the tip portion 24 is touching the internal wall or the like in a body cavity with a pressure at an appropriate value or more.

And when the state management portion 81 determines that it is not touching with a pressure at the appropriate value or more, the routine goes on to the next step S48, it is determined whether a target position corresponding to the angle command value is reached or not by a detected value of the encoder, and if the target position is not reached, the routine returns to Step S46, while the target position is reached, the control processing for this angle operation is finished.

On the other hand, if the state management portion 81 determines at Step S47 that touching is made with a pressure at the appropriate value or more, the control processing for the angle operation is finished without the processing at the next Step S48.

When the angle operation is carried out in this way, the state management portion 81 performs control processing so that the curved portion 27 is curved to the target position corresponding to the command value by the angle operation. If the tip portion 24 touches the internal wall or the like of the body cavity with a pressure larger than a set value, the control is performed so that further curving is restrained.

Therefore, at insertion of the insertion portion 21 into the body cavity, even if the user tries to insert it along the bent pipeline in the angle operation, contact with a pressure larger than the set value can be avoided, which can reduce a pain to the patient and also realize smooth insertion.

Control may be made so that the rigidity is further changed by the actuator for rigidity changing according to detected output of the touch sensor 142.

Next, the control processing of the rigidity changing operation will be described referring to Fig. 22. This control processing is basically the same as that in Fig. 21.

When the control processing of the rigidity changing operation is started, as shown in Step S51, the state management portion 81 determines if the rigidity changing control is effective or not.

Specifically, as shown in Fig. 16B, a rigidity of the insertion portion is allocated to the scope switches SW1 to SW5 by the main menu, and the state management portion 81 determines if the scope switch of insertion portion is pressed for the rigidity to become effective.

And when the state management portion 81 determines that the rigidity changing control is not effective, the routine moves to Step S55 to maintain the previous command value. On the other hand, if it is determined that the rigidity changing control is effective, the routine goes on to the next Step S52, and the state management portion 81 obtains the state data by operation of the track ball 69.

And at the next Step S53, the state management portion 81 further determines if there is a state change or not on the basis of the output of the track ball displacement detection portion 95.

In this case, if the state management portion 81 determines that there is no state change, the routine moves to Step S55, while if it is determined that there is a state change, the command value corresponding to the rotating direction and rotating amount of the track ball 69 is calculated at the next Step S54.

After processing of step S54 or S55, as shown in Step S56, the state management portion 81 sends the command value to the actuator driving portion 94 through the rigidity changing control portion 93 and performs servo processing of the rigidity changing actuator 54A or 54B.

That is, the actuator driving portion 94 drives the actuator rigidity changing 54A or 54B so as to obtain target rigidity corresponding to the command value on the basis of the command value. At that time, the rigidity changing actuator 54A or 54B detects the rigidity changing state by the encoder 54c, and the actuator driving portion 94 drives the rigidity changing actuator 54A or 54B so that a value detected by this encoder 54c reaches the target rigidity.

At Step S57 during such servo processing, the rigidity changing control portion 93 or the state management portion 81 determines if the rigidity changing actuator 54A or 54B is within a changeable range by the actuator driving portion 94, and if this variable range is deviated, this rigidity changing control processing is finished.

Also, at step S57, if the rigidity changing actuator 54A or 54B is within the changeable range, further at the next Step S58, the rigidity changing control portion 93 or the state management portion 81 determines if the target rigidity is reached or not, and if the target rigidity is not reached, the routine returns to step S56 and the servo processing is continued. If the target rigidity is reached in this way, the rigidity changing control processing is finished.

Also, the UPD unit 76 detects by the UPD coil unit 8 the position of the UPD coil 58 arranged inside the insertion portion 21 of the endoscope 3, calculates the insertion shape of the insertion portion 21 and displays the insertion portion shape, that is, the UPD image on the display screen of the observation monitor 6.

Figs. 23A to 23D show states that the menu screen on the right corresponds to the UPD image on the left, respectively, and when the rigidity of the rigidity changing actuators 54A, 54B is selected/set by a user on the menu screen, by displaying the rigidity portions of the rigidity changing actuators 54A, 54B provided at a plurality of locations (two locations in the specific example) in color corresponding to set rigidity, the rigidity of that portion is made identifiable easily.

Fig. 23A shows a display state of the main menu and that the user selects the insertion portion rigidity changing in this display state. In this case, since it is immediately before the insertion portion rigidity changing is selected, the UPD image displays sections A, B of the actuators 54A, 54B for rigidity changing in the manner not distinguished from the portions other than these sections A, B.

When the insertion portion rigidity changing is selected as in Fig. 23B, rigidity section ranges to be set for the sections A, B of the rigidity changing actuators 54A, 54B at two locations are shown, and the screen becomes a rigidity setting screen for setting a rigidity from the low rigidity (flexible) state to the high rigidity state in the sections A, B with the current rigidity positions shown by circles. In this case, flexible to rigid are displayed in different colors, respectively.

Therefore, in the corresponding UPD image, a part of the rigidity changing actuator is displayed in color, which is the display color corresponding to rigidity to which the rigidity changing actuator is set. In the state in Fig. 23B, the rigidity section is set close to the flexible state, and the sections A, B portions of the actuators 54A and 54B for rigidity changing in the UPD image in this case are displayed in yellow.

Fig. 23C shows a case where the rigidity of the section B of the rigidity changing actuator 54B is set to rigidity close to the center in the state of Fig. 23B, for example, and the section B of the rigidity changing actuator 54B in the UPD image in this case is displayed in green.

Also, Fig. 23D shows a case where the rigidity of the section B of the rigidity changing actuator 54B is set to high rigidity (harder value) in the state of Fig. 23B or Fig. 23C, for example, and the section B of the rigidity changing actuator 54B in the UPD image in this case is displayed in blue.

By displaying in this way, the user can freely set the rigidity of the rigidity changing actuators 54A, 54B, and since the sections A, B of the set rigidity changing actuators 54A, 54B are shown in the display color corresponding to the set rigidity, the user can easily identify the rigidity of the rigidity changing actuators 54A, 54B.

Also, since the shape of the insertion portion 21 is displayed by the UPD coil 58, the operator can easily perform insertion work and the like of the insertion portion 21.

Next, the processing content on the endoscope 3 side and the endoscope system control portion 5 side of a human interface realizing the remote control operation by the user will be described referring to Figs. 24 and 25. In Figs. 24 and 25, the human interface is abbreviated as HMI.

When the processing of the human interface is started as shown in Fig. 24, the state management portion 81 waits for the angle effective switch to be turned OFF. That is, it waits for the angle effective switch to be turned OFF by pressing of the track ball 69.

And when the angle effective switch is turned OFF, the state management portion 81 issues a GUI (Graphical User Interface) display message as shown in the next Step S62. This GUI display message is sent from the endoscope 3 through the AWS unit 4 to a control CPU in the system control unit 117 in the endoscope system control device 5 wirelessly.

After the GUI display message is issued, the state management portion 81 is brought into the state waiting for receiving of the GUI display completion message from the endoscope system control device 5 in the next Step S63. And if the state management portion 81 can not receive this GUI display completion message, it goes on to Step S64 and determines if the condition to end retry is applicable or not. If the retry end condition is not applicable, the routine returns to step S63, while the retry end condition is applicable, the routine ends in error.

When the state management portion 81 receives the display completion message in the processing at step S63, the routine moves to Step S65, where it is determined whether the angle effective switch is turned ON or not. If the angle effective switch is turned ON, the state management portion 81 issues the GUI end message as shown in Step S66.

This GUI end message is sent to the endoscope system control device 5 wirelessly from the endoscope 3 through the AWS unit 4 as with the GUI display message. And after this GUI end message is issued, the state management portion 81 is brought into the state waiting for receiving of the GUI display end message from the endoscope system control device 5 at the next step S67. And when the state management portion 81 receives the GUI display end message, this human interface processing is ended.

On the other hand, if the state management portion 81 can not receive the GUI display end message, the routine goes on to Step S68, where it is determined whether the retry end condition is applicable or not. If the retry end condition is not applicable, the routine returns to Step S66, while if the retry end condition is applicable, the routine ends in error.

Also, at Step S65, if the angle effective switch is not turned ON, the routine moves to the processing on the menu screen at Step S69, and at this Step S69, the state management portion 81 determines whether the state of the track ball 69 has been changed or not by determining whether there is a change amount more than a threshold value from the output of the track ball displacement detection portion 95.

And as shown in Step S70, if the state management portion 81 determines that the state of the track ball 69 has been changed, the state data (change data) of the track ball 69 is obtained.

In this case, the user can instruct to select a function of a desired item by a cursor moving in correspondence with the operation of the track ball 69 on the main menu screen of Fig. 16B.

And as shown in Step S71, the state management portion 81 sends the state data corresponding to the operation of the track ball 69 by the user. This state data is sent as packet data from the endoscope 3 through the AWS unit 4 to the endoscope system control device 5 in synchronization with the image pickup data of the CCD 25. After sending of this state data, the routine returns to the processing of Step S65.

When the state management portion 81 determines that there is no state change of the track ball 69 at Step S69, it is determined whether there is change in switch state (Switches SW1 to SW5) as shown in Step S72 by detection output by the switch pressing detection portion 96.

At this Step S72, if it is determined that there is no switch state change, the routine returns to Step S65, while if it is determined that there is switch state change, the state management portion 81 obtains switch pressing state data as shown in Step S73 and further sends the switch pressing data obtained in the next Step S74 and returns to the processing in Step S65.

On the other hand, when the processing of the human interface is started as shown in Fig. 25, the CPU of the system control unit 117 of the endoscope system control device 5 is brought into the state waiting for receiving of the GUI display message from the endoscope 3 in the first Step S81. This CPU waits for receiving of the GUI display message wirelessly through the sending/receiving unit 101 in Fig. 8 or Fig. 14.

And as shown in Step S82, when the CPU of the system control unit 117 receives the GUI display message, it performs control processing of the GUI display. That is, the CPU performs GUI display control for the image processing unit 116.

After the GUI display processing in Step S82, the CPU issues the display completion message as shown in Step S83. The CPU sends this display completion message through the sending/receiving unit 101. At the next Step S84, the CPU determines whether the GUI end message has been received or not from the endoscope 3 side. And when this GUI end message has been received, the CPU performs processing to end the GUI display in Step S85 and at the next Step S86, it issues the GUI display end message and ends this human interface processing.

If the GUI end message has not been received in Step S84, the routine moves to step S87 and the CPU determines whether there is a change in received data of the track ball 69. The determination of change in the received data of the track ball 69 is made upon receipt of a determination result of the state change of the track ball 69 on the endoscope 3 side. And if there is a change in the received data, as shown in Step S88, the state data of the track ball 69 is obtained. Moreover, at the next Step S89, the CPU moves the cursor by a moving amount corresponding to the obtained state data (change data) of the track ball 69. And the routine returns to the processing of Step S84.

Also, in the processing of Step S87, if it is determined that there is no change in the received data of the track ball 69, the CPU determines whether there is change in the received data of the switch as shown in Step S90 based on the received data received as the sent data of the determination result on the endoscope 3 side.

If it is determined that there is a change in the received data of the switch, the CPU obtains the switch pressing state data form the sending information from the endoscope 3 as shown in Step S91. Moreover, as shown in Step S91, the CPU performs the processing executed by the function allocated to the pressed switch and returns to the processing of Step S84. Also, if there is no change in the received data of the switch at Step S90, the routine returns to the processing of Step S84.

According to the endoscope 3 of this embodiment constituting the endoscope system 1 performing such operations, this endoscope 3 is made capable of separation to the endoscope main body 18 and the tube unit 19 in the operation portion 22 to make the tube unit 19 disposable so that washing, disinfection and the like of the endoscope main body 18 can be performed easily.

That is, in the air/water supply pipeline 60a and the suction pipeline 61 a in the endoscope main body 18, a universal cable corresponding to the tube unit 19 can be made extremely shorter than the conventional case of integral formation, which makes washing and disinfection easy.

Also, in the conventional case where the universal cable corresponding to the tube unit 19 is integrally formed, the universal cable is provided adjacent to the operation portion 22 in the bent state, but in this embodiment, it becomes the slightly bent pipeline connector portion 51a in the connector portion 51 of the operation portion 22, while the other portion is the air/water supply pipeline 60a and the suction pipeline 61a extending substantially straight. Thus, operations such as washing, disinfection, drying and the like inside the pipeline can be carried out easily and in a reduced time. Therefore, the setup for the state capable of endoscope inspection can be set in a reduced time.

Moreover, since this embodiment is in the structure that the endoscope main body 18 and the tube unit 19 are freely detachably connected in the contactless manner, even if washing and disinfection of the endoscope main body 18 is repeated, defective conducting or the like of the contact is not generated as in the otherwise contact case, and the reliability can be improved.

Furthermore, in this embodiment, many operating means such as angle operating means, air/water supply operating means, suction operating means, rigidity changing means, freeze operating means, release operating means and the like are provided at the operation portion 22 and these operating means are controlled in the intensive (centralized) manner by the control circuit 57 provided in the operation portion 22. Also, this control circuit 57 is configured to intensively control the light emitting means for emitting illumination light for image capturing and the image pickup means for image capturing together with the above operating means.

In this way, in this embodiment, since intensive control of various functions provided at the endoscope main body 18 is performed by the control circuit 57 provided inside the operation portion 22 and various functions for the operating means of the AWS unit 4 connected to the endoscope body 18 and the endoscope system control device 5 for sending/receiving information wirelessly are also controlled in the intensive manner, the user (more specifically, operator) can freely carry out various operations by the various operating means provided at the operation portion 22, which can drastically improve operability.

Particularly in this embodiment, since by providing the control circuit 57 for intensive control in the operation portion 22, the image data obtained by image capturing by the CCD 25 and the various signals by the operating means are made into packet and transferred from this control circuit 57 in common by a pair signal lines 71b, the number of electric signal lines can be reduced (specifically, the number of lines can be reduced to two signal lines for transferring a signal and two power lines for transferring power. Also, if one of the signal lines and the power lines are used in common, the total number of lines can be made to three).

Therefore, the number of signal lines required to be inserted into the tube unit 19 connected at the connection portion in the operation portion 22 can be also reduced, which makes possible to have the tube unit 19 disposable.

Also, by reducing the number of signal lines inserted into the tube unit 19, the tube unit 19 can be reduced in diameter and can be bent easily, which improves operability when being operated by the user.

As a variation of the endoscope system 1 shown in Fig. 4, an endoscope system 1 B in the configuration as shown in Fig. 26 may be provided.

This endoscope system 1B is so configured that, in the endoscope system 1 in Fig. 4, the AWS unit 4 is housed in a recess portion provided on the side face of the laying table 2a of the inspection bed 2. Specifically, it is so configured that the AWS unit 4 is housed in the vicinity of the housing-recess portion 2b provided at the laying table 2a of the inspection bed 2.

In this AWS unit 4, the sending/receiving unit 77 which performs sending/receiving in wireless manner as shown in Fig. 8 is provided on the upper face, for example. Also, the scope connector 40 is provided on the front face exposed outward when being housed in the recess portion so that the scope connector 41 of the endoscope 3 can be freely detachably connected. That is, the AWS unit 4 is configured so that the scope connector 40 is arranged at the position where the scope connector 40 for connecting the scope connector 41 of the endoscope 3 is exposed outward when being housed in the recess portion of the laying table 2a.

Thus, as shown by a two dotted chained line in Fig. 26, a patient lies down with the lower body faced to the UPD coil unit 8 side for a colon inspection, for example. Therefore, when the insertion portion is pulled out of the body cavity of the patient on the UPD coil unit 8 after completion of the inspection, the operator can pull out the scope connector 41 from the AWS unit 4 in the vicinity of the UPD coil unit 8, puts the endoscope in the scope tray 39, and can carry the endoscope after use to a washing area by pushing the tray-carrying trolley 38 with the tray 39 loaded thereon, which makes tidying easy. Particularly, since the lower body, the AWS unit 4 and the scope tray 39 are located close to each other and the distance to move the endoscope 3 to the scope tray 39 is reduced, the contaminated endoscope can be tidied immediately after use. Moreover, the endoscope after use can be housed in the scope tray 39 on the spot, moved to the washing area and the scope tray 39 containing the endoscope can be washed altogether in the washing area without touching the endoscope and returned to the inspection bed 2. Thus, washing and handling performance of the endoscope is extremely improved.

Moreover, since the AWS unit 4 is provided at the laying table 2a, the operator can handle the endoscope easily when operating it from various directions around the inspection bed 2.

The other configurations are the same as in Fig. 4. In the case of this configuration, since the AWS unit 4 is mounted to the inspection bed 2 when performing an endoscope inspection or the like with the endoscope 3, it can be connected to the AWS unit 4 without extending the tube unit 19 extended from the endoscope 3 long, which can provide an environment where the operator can operate the endoscope easily. The other effects are the same as the case of the endoscope system 1 in Fig. 4.

According to the present invention, the medical bed can be provided in which the endoscope after use can be tidied easily.

## Claims

1. A medical bed comprising:
a laying table for inspection or treatment by an endoscope, on which a patient is laid; and
a tray housing portion for housing an endoscope housing tray in which the endoscope is housed.

2. The medical bed according to claim 1, further comprising a trolley for carrying tray loaded with the endoscope housing tray, for housing the endoscope housing tray in the tray housing portion and carrying it out of the tray housing portion.

3. The medical bed according to claim 1, further comprising an air/water supply and suction device provided in the vicinity of the tray housing portion of the laying table, for supplying and suctioning air/water for the endoscope.

4. The medical bed according to claim 2, further comprising an air/water supply and suction device provided in the vicinity of the tray housing portion of the laying table, for supplying and suctioning air/water for the endoscope.

5. The medical bed according to claim 3, wherein the air/water supply and suction device is housed in a recess portion of the laying table; and
the tray housing portion is provided in the vicinity of the recess portion.

6. The medical bed according to claim 4, wherein the air/water supply and suction device is housed in a recess portion of the laying table; and
the tray housing portion is provided in the vicinity of the recess portion.

7. The medical bed according to claim 5, wherein the air/water supply and suction device has a connector for connecting the endoscope arranged at a position where the connector is exposed outward when the device is housed in the recess portion of the laying table.

8. The medical bed according to claim 6, wherein the air/water supply and suction device has a connector for connecting the endoscope arranged at a position where the connector is exposed outward when the device is housed in the recess portion of the laying table.

9. The medical bed according to claim 3, wherein the laying table further has a an endoscope insertion shape detecting unit, and the tray housing portion and the air/water supply and suction device are provided in the vicinity of the unit for detecting the endoscope insertion shape of the laying table.

10. The medical bed according to claim 4, wherein the laying table has a unit for detecting the endoscope insertion shape, and the tray housing portion and the air/water supply and suction device are provided in the vicinity of the unit for detecting the endoscope insertion shape of the laying table.

11. The medical bed according to claim 5, wherein the laying table has a unit for detecting the endoscope insertion shape, and the tray housing portion and the air/water supply and suction device are provided in the vicinity of the unit for detecting the endoscope insertion shape of the laying table.

12. The medical bed according to claim 6, wherein the laying table has a unit for detecting the endoscope insertion shape, and the tray housing portion and the air/water supply and suction device are provided in the vicinity of the unit for detecting the endoscope insertion shape of the laying table.

13. The medical bed according to claim 7, wherein the laying table has a unit for detecting the endoscope insertion shape, and the tray housing portion and the air/water supply and suction device are provided in the vicinity of the unit for detecting the endoscope insertion shape of the laying table.

14. The medical bed according to claim 8, wherein the laying table has a unit for detecting the endoscope insertion shape, and the tray housing portion and the air/water supply and suction device are provided in the vicinity of the unit for detecting the endoscope insertion shape of the laying table.
